# EUROPEAN PATENT SPECIFICATION

(11) **EP 2 199 411 B1**
(45) Date of publication and mention of the grant of the patent: **06.05.2015**
(21) Application number: 08021838.1
(22) Date of filing: 16.12.2008
(51) Int. Cl.: C12Q 1/68

(54) **Epigenetic markers for the identification of CD3 positive T-lymphocytes**
Epigenetische Marker zur Identifikation von CD3+ T-Lymphozyten
Marqueurs épigénétiques pour l'identification des lymphocytes T CD3+

(43) Date of publication of application: 23.06.2010
(73) Proprietor: Epiontis GmbH, 12489 Berlin (DE)
(72) Inventor: Olek, Sven, 12207 Berlin (DE); Türbachova, Ivana, 12207 Berlin (DE)
(74) Representative: Krauss, Jan

(56) References cited:
- FLANAGAN B F ET AL: "DNASE HYPERSENSITIVITY AND METHYLATION OF THE HUMAN CD3G AND D GENES DURING T-CELL DEVELOPMENT" IMMUNOGENETICS, SPRINGER VERLAG, BERLIN, DE, vol. 31, no. 1, 1 January 1990 (1990-01-01), pages 13-20, XP009113698 ISSN: 0093-7711
- CLEVERS H ET AL: "AN ENHANCER LOCATED IN A CPG-ISLAND 3' TO THE TCR-CD3-EPSILON GENE CONFERS T LYMPHOCYTE-SPECIFICITY TO ITS PROMOTER" EMBO (EUROPEAN MOLECULAR BIOLOGY ORGANIZATION) JOURNAL, vol. 8, no. 9, 1989, pages 2527-2536, XP002519563 ISSN: 0261-4189
- TUTT LANDOLFI M M ET AL: "Specific demethylation of the CD4 gene during CD4 T lymphocyte differentiation." MOLECULAR IMMUNOLOGY JAN 1997, vol. 34, no. 1, January 1997 (1997-01), pages 53-61, XP002519564 ISSN: 0161-5890
- LUSSO P ET AL: "Induction of CD4 and susceptibility to HIV-1 infection in human CD8<+> T lymphocytes by human herpesvirus 6" NATURE 1991 GB, vol. 349, no. 6309, 1991, pages 533-535, XP002519565 ISSN: 0028-0836
- GEORGOPOULOS K ET AL: "A T cell-specific enhancer is located in a DNase I-hypersensitive area at the 3' end of the CD3-delta gene." THE EMBO JOURNAL AUG 1988, vol. 7, no. 8, August 1988 (1988-08), pages 2401-2407, XP002519566 ISSN: 0261-4189

## Description

The present invention relates to a method, in particular an *in vitro* method, for identifying CD3CD4 and/or CD3CD8 positive T lymphocytes of a mammal, based on the methylation status in the T-lymphocyte specific methylated regions (TLSDRs) in the CD3 gamma-delta intergenic region, wherein a demethylation of at least one CpG in the analyzed sample to at least 90% is indicative for memory and naive CD4⁺ T lymphocytes and memory and naive CD8⁺ T lymphocytes. The method may further comprise analyzing the methylation status of at least one CpG position in the genes SLA2, CHRNA3, C16orf24, LCK, FASLG, CD7, SIT1, IL32, CXCR6, UBASH3A, GRAP2, ITGB7 and TXK which also allows the unambiguously identification of all CD3 positive T lymphocytes. For further unambiguous identification of all CD8 cells, also the equivalent analysis GNGT2, CRTAM, IL2RB and ZBTB32 can be employed. Among the CD3 positive T lymphocytes, these markers are capable to segregate between CD8 and CD4 positive cells. Equivalently, FLJ00060, FLJ38379, PPP6C, CD226, ZBTB7B and TNFAIP8 are capable of positively identifying CD4 expressing cells in whole blood and segregate between CD4 and CD8 positive CD3 positive cells.

Furthermore, the present invention relates to the use of a kit for performing the above methods. The present invention allows for a precise analysis even from sub-optimal quality samples, such as non-freshly obtained blood or serum samples. It is one aim of this invention to provide a novel, more robust means to quantitatively detect and measure particular subsets of the blood within any solid organs or any body fluid of a mammal.

Employing this method, the inventors provide for novel, not previously known means of determining, quantitating and routinely measuring T lymphocytes.

### Background of the invention

T-lymphocytes are a major component of the mammalian immune system. Both CD4 and CD8 T-cells are responsible for proper functioning of said immune system. Whereas CD8 T-cells mediate the cytotoxic immune defence, CD4 cells - the so called helper T-cells - assist both the humoral and the cell mediated immune defence. The heterodimeric T-cell antigen receptor (TCR) is bound to a monomorphic protein complex called CD3. CD3 consists of subelements CD3 γ, -δ, and -ε, and is expressed on all peripheral T-cells, but only on a subset of the thymocytes. The genes for CD3 γ, -δ, and -ε are encoded on neighbouring loci on chromosom 11 (11q23).

Role of CD3 in signal transduction - The function of the CD3-chains is both the formation and the transport of the full CD3 complex to the cell surface, as well as the signal transduction upon stimulation through the heterodimeric T-cell antigen receptor. The amino acid sequence of the cytoplasmatic part of CD3γ, -δ, -ε each contains a motif that becomes phosphorylated upon stimulation, and thus activated. This so called ITAM (immunoreceptor tyrosine-based activated motif) serves as docking point for different tyrosine kinases. Individuals with defective CD3γ or-ε-chains suffer from a severe clinical autoimmune deficiency.

Even though almost all cells in an individual contain the exact same complement of DNA code, higher organisms must impose and maintain different patterns of gene expression in the various types of tissue. Most gene regulation is transitory, depending on the current state of the cell and changes in external stimuli. Persistent regulation, on the other hand, is a primary role of epigenetics - heritable regulatory patterns that do not alter the basic genetic coding of the DNA. DNA methylation is the archetypical form of epigenetic regulation; it serves as the stable memory for cells and performs a crucial role in maintaining the long-term identity of various cell types.

The primary target of methylation is the two-nucleotide sequence Cytosine-Guanine (a 'CpG site'); within this context cytosine (C) can undergo a simple chemical modification to become 5-methyl-cytosine. In the human genome, the CG sequence is much rarer than expected, except in certain relatively dense clusters called 'CpG islands'. CpG islands are frequently associated with gene promoters, and it has been estimated that more than half of the human genes have CpG islands (Antequera and Bird, Proc Natl Acad Sci USA 90: 11995-9, 1993). Aberrant methylation of DNA is frequently associated with the transformation from healthy to cancerous cells. Among the observed effects are genome-wide hypomethylation, increased methylation of tumour suppressor genes, and hypomethylation of many oncogenes (reviewed, for example, by Jones and Laird, Nature Genetics 21:163-167, 1999; Esteller, Oncogene 21:5427-5440, 2002; and Laird, Nature Reviews/Cancer 3:253-266, 2003). Methylation profiles have been recognized to be tumour specific (i.e., changes in the methylation pattern of particular genes or even individual CpGs are diagnostic of particular tumour types), and there is now an extensive collection of diagnostic markers for bladder, breast, colon, oesophagus, stomach, liver, lung, and prostate cancers (summarized, for example, by Laird, Nature Reviews/Cancer 3:253-266, 2003).

Hamerman et al. (in: Hamerman JA, Page ST, Pullen AM. Distinct methylation states of the CD8 beta gene in peripheral T cells and intraepithelial lymphocytes. J Immunol. 1997 Aug 1;159(3):1240-6) distinguish between CD4 and CD8 T-lymphocytes.

EP 1 213 360 describes a method of identifying a cell, tissue or nucleus, comprising collecting information on the methylation pattern of DNA isolated from the cell, tissue or nucleus and analyzing the resultant information.

WO 2004/050706 describes a sub-group of T-cells, and relates to characteristics of regulatory T-cells which define them as such. The application also describes the uses of such T-cells, compositions comprising them, and chemokines which recruit them in the modulation of an immune response.

Flanagan et al (Immunogenetics, vol.3 1, pp. 13-20, 1990) discloses hypomethylation of the CD3 gamma-delta region in some T-cells.

Finally, EP 1 826 279 describes a method, in particular an *in vitro* method, for identifying FoxP3-positive regulatory T cells, preferably CD25⁺ CD4⁺ regulatory T cells of a mammal, comprising analyzing the methylation status of at least one CpG position in the gene foxp3 or an orthologous or paralogous gene thereof, and the use of DNA-methylation analysis of the gene of the transcription factor FoxP3 for a detection and quality assurance and control of regulatory T cells. While the measurement and determination of CD4 and CD8 cells is generally easy and is usually achieved through analyzing the expression of said antigens on the cellular surface, clinically, it remains challenging to determine these cell types, since for the commonly used FACS analysis the cell samples need to be freshly isolated or immediately fixated in order to keep the cell entities intact. Thus, the detection of T lymphocytes, while desirous, is problematic, particularly for routine applications.

In view of the above, it is an object of the present invention to provide an improved and in particular robust method based on DNA methylation analysis as a superior tool in order to more conveniently and reliably identify T-lymphocytes.

The present invention solves the above object by providing a method for identifying CD3⁺CD4⁺ and/or CD3⁺CD8⁺ naive and/or memory T-lymphocytes in a sample derived from a mammal comprising T cells, comprising analyzing the methylation status of at least one CpG position in the T-lymphocyte specific demethylated region (TLSDR) located in amplicon 1405 (SEQ ID No.6), amplicon 1406 (SEQ ID No.7) and/or amplicon 1408 (SEQ ID No.8), and identifying CD3 CD4 and/or CD3⁺CD8⁺ naive and/or memory T-lymphocytes based on said methylation status, wherein a demethylation of at least one CpG position to at least 90% in said amplicon is indicative for a CD3⁺ CD4⁺, and/or CD3⁺ CD8⁺ naive and/or memory T-lymphocyte cell.

The present invention is based on the surprising finding of the inventors that the identification of said TLSDRs in the CD3 gene as a specific epigenetic marker can greatly facilitate the clinical routine application of the analysis of the above markers. In contrast to FACS and mRNA measurements, the respective measurement(s) can be done independent of purification, storage and to quite some extend also to tissue quality.

In another preferred embodiment of the method according to the present invention, said at least one CpG position in said sample is demethylated to more than 91 % and preferably more than 92% and most preferred more than 95%.

This concept is based on specific demethylation of the TLSDR regions in CD3 positive T-lymphocytes. Using a simple and precise quantitative PCR method, the inventors show that CD3 demethylation in said regions represents a surrogate marker for these T-lymphocyte counts in blood or tissues. The present inventors have thus identified particular regions within the CD3 gene, which are functionally involved in, or mandatory associated with, the existence of CD3 positive T-lymphocytes.

The regions are the TLSDRs within the nucleotide sequence according to SEQ ID No.1 containing CpG motifs, which display a differential methylation status when cells expressing CD3 in either CD4⁺ or CD8⁺ cells compared with all other cells, not expressing CD3 if, for example, the bisulphite sequencing method is used.

The inventors could demonstrate that in said CD3⁺ cells the CpG motifs are almost completely demethylated (i.e. to more than 70%, preferably 80%, preferably, more than 90% and most preferred more than 95%), whereas the same motifs are completely methylated in all CD3⁻cells. The differential methylation of the CpG motifs within the aforementioned region correlates with CD3 expression. Thus, determination of the methylation status of the TLSDRs in the *CD3* locus could become a valuable tool to identify T-lymphocytes, such as will be required/or at least of some value for measuring T-lymphocytes in autoimmune diseases, transplant rejections, cancer, allergy, or just the T-lymphocytes related immune status in any envisionable context, when desired. The assays allows measurement if T-lymphocytes without purification or any staining procedures. It even reports in solid tumors or other solid tissues the number of cells demethylated in said region, thus showing the total amount of CD3 positive tumor infiltrating T-lymphocytes.

The inventors have shown that the potential for constitutive expression of CD3 in T-lymphocytes coincides with epigenetic, i.e., DNA methylation based regulation. DNA methylation is a biologically and chemically stable epigenetic modification, resulting in long-term gene expression changes. The inventors found demethylation at the human CD3 locus to be restricted to T-lymphocytes when tested against all major peripheral blood cell types and a selection of non-blood cells. These data indicated that epigenetic modifications in the CD3 locus serve as valuable marker for the identification of cells with the phenotype of T-lymphocyte, regardless of the expression of the specific delta or gamma sub-chains. In another preferred aspect of the method according to the present invention the methylation status of at least one CpG position in the genes SLA2, CHRNA3, C16orf24, LCK, FASLG, CD7, SIT1, IL32, CXCR6, UBASH3A, GRAP2, ITGB7 and TXK is additionally analysed in analogy to what is described herein for CD3. These genes thus also allow the unambiguous identification of all CD3 positive T lymphocytes. Thus, in a preferred embodiment of the method according to the present invention, said at least one CpG position is present in the 5' region upstream from the transcription start, promoter region, intron, and/or exon/intron border within the gene(s) SLA2, CHRNA3, C16orf24, LCK, FASLG, CD7, SIT1, IL32, CXCR6, UBASH3A, GRAP2, ITGB7 and TXK.

In order to further unambiguously identify all CD8 cells, the present invention in another preferred aspect thereof provides the equivalent analysis of the genes GNGT2, CRTAM, IL2RB and ZBTB32 among the CD3 positive T lymphocytes, as these markers are capable to segregate between CD8 and CD4 positive cells. This analysis is preferably performed simultaneously or subsequently to the analysis for the CD3 phenotype of the T-lymphocytes. Equivalently, FLJ00060, FLJ38379, PPP6C, CD226, ZBTB7B and TNFAIP8 are capable of positively identifying CD4 expressing cells in whole blood and segregate between CD4 and CD8 positive CD3 positive cells.

The present invention provides the surprising finding that in particular regions of the gene for CD3,
the so-called "TLSDRs" (T lymphocyte specific demethylated regions), the CpG motifs are almost completely demethylated (i.e. to more than 90%, preferably 91%, preferably, more than 92% and most preferred more than 95%), whereas the same motifs are completely methylated in all non T lymphocytes. Thus, these regions and the diagnostic uses thereof also provides a valuable and reliable tool for a diagnostic analysis according to the present invention. The TLSDR according to the present invention are located in amplicon No. 1405 (SEQ ID No. 6), amplicon No. 1406 (SEQ ID No. 7), and/or amplicon No. 1408 (SEQ ID No. 8). All of these amplicons are parts of the overall region of interest depicted in SEQ ID No.1.

In a preferred embodiment of the method according to the present invention, said analysis of the methylation status comprises amplification with at least one primer of suitable primer pairs that can be suitably designed based on SEQ ID No. 1, preferably oligomers according to any of SEQ ID No. 2 to 5.

Preferably, the amplification involves a polymerase enzyme, a PCR or chemical amplification reaction, or other amplification methods as known to the person of skill as described below, e.g. in the context of MSP, HeavyMethyl, Scorpion, MS-SNUPE, MethylLight, bisulfite sequencing, methyl specific restriction assays. With the amplification, the amplicon of the TLSDR or any other region in the CD3 gene or any paralog or ortholog as described herein is produced that is a particularly preferred "tool" for performing the method(s) according to the present invention. Consequently, an oligomer according to any of SEQ ID No. 2 to 5 or the amplicon as amplified by a primer pair as mentioned above constitute preferred embodiments of the present invention.

The person of skill will furthermore be able to select specific subsets of CpG positions in order to minimise the amount of sites to be analyzed, for example at least one of CpG position 1, 2, 3, 4, 5, 6, 7, 8, 9, and 10 of the amplicon No. 1405 (SEQ ID No. 6), amplicon No. 1406 (SEQ ID No. 7), and amplicon No. 1408 (SEQ ID No. 8).

The positions are numerically counted from the 5'-end of the amplicon as generated and analysed. Preferred are combinations of 4, 5, 6, or 7 positions, which are producing enough information in order to be informative in the context of the present invention.

In order to analyze the methylation status of CpG positions, any known method to analyse DNA methylation can be used. In a preferred embodiment of the method according to the present invention, the analysis of the methylation status comprises a method selected from methylation specific enzymatic digests, bisulphite sequencing, analysis selected from promoter methylation, CpG island methylation, MSP, HeavyMethyl, MethyLight, Ms-SNuPE or other methods relying on a detection of amplified DNA. These methods are well known to the person of skill, and can be found in the respective literature.

In a preferred embodiment of the method according to the present invention, said method is suitable for routine application, for example on a DNA-chip. Based on the above information and the respective literature, the person of skill will be able toadjust the method a above to such settings.

In another preferred embodiment of the method according to the present invention, the identification comprises a distinction of said T-lymphocytes from all major peripheral blood cell types or non-blood cells, preferably, but not limited to, CD14, CD15,CD19, and CD56.

In yet another preferred embodiment of the method according to the present invention, the sample is selected from a mammalian body fluid, including human blood samples, or a tissue, organ or cell type blood sample, a sample of blood lymphocytes or a fraction thereof. Preferably, said mammal is a mouse, rat, monkey or human. The samples can be suitably pooled, if required.

Another preferred aspect of the method according to the present invention then further comprises the step of concluding on the immune status of said mammal based on said T-lymphocytes as identified. The general lymphocyte population can be quantified and either be used as a benchmark to relatively quantify further detailed subpopulations such as the CD3CD4CD25 positive regulatory T cells (als bezugspunkt) or it can be used to finally detect this population to determine the overall immune activity status.

In yet another preferred embodiment of the methods according to the present invention, the mammal suffers from or is likely to suffer from autoimmune diseases, transplant rejections, cancer, and/or allergy.

Yet another preferred aspect of the present invention then relates to the use of a kit for identifying and/or monitoring CD3⁺ CD4⁺, and/or CD3⁺ CD8⁺ T-lymphocytes in a mammal according to the method of present invention as described herein. Said kit comprises a) a bisulfite reagent, and b) materials for the methylation analysis of CpG positions as comprised by the amplicon No. 1405 (SEQ ID No. 6), amplicon No. 1406 (SEQ ID No. 7), and amplicon No. 1408 (SEQ ID No. 8). The positions consist of positions 1, 2, 3, 4, 5, 6, 7, 8, 9, and 10 of amplicon 1405, CpG positions 1, 2, 3, 4, 5, 6, 7 and 8 of amplicon 1408 and CpG positions 1, 2, 3, 4, 5, 6 and 7 of amplicon 1408. Most preferred are 5, 6, or 7 or all positions on said amplicon.

In summary, using the CD3 marker, the inventors very specifically identified (but not differentiated) both CD3/CD4 positive as well as CD3/CD8 positive naive and memory T lymphocytes. Using the marker CD8beta, for example CD8 positive lymphocytes could then be distinguished from CD4 lymphocytes. This, when using a combination of the present marker(s) and the CD8beta marker, CD4 and CD8 cells can be specifically distinguished. This was not possible before the invention, since all CD4 looked identical to non-T lymphocytes.

The invention will now be further described based on the following examples and with reference to the accompanying figures and the sequence protocol, without being limited thereto. In the Figures and Sequences,
Figure 1 shows the differentially methylated gene region as found for CD3 g and d and are indicated by red lines as "blast hits".

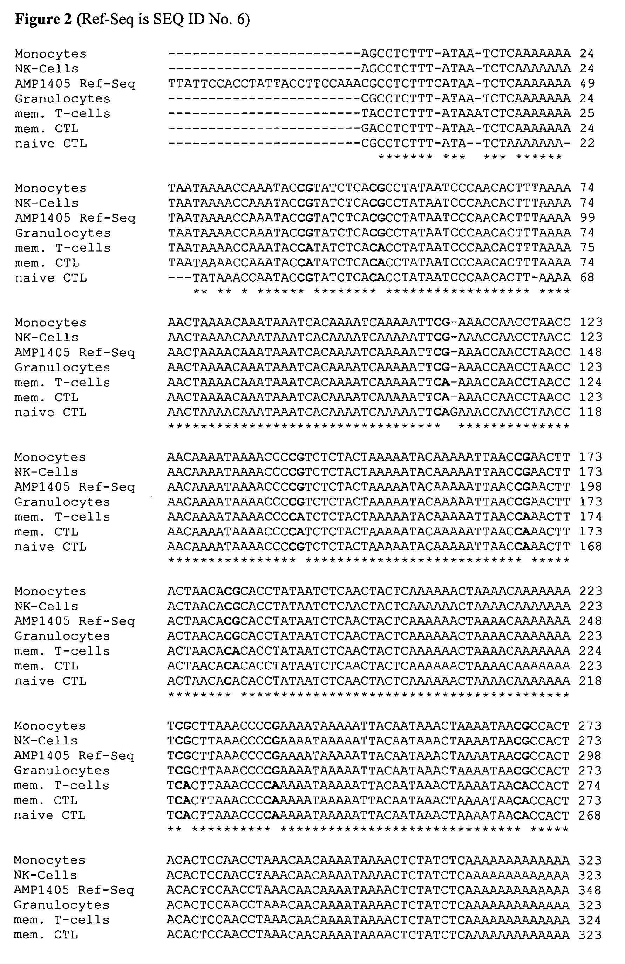

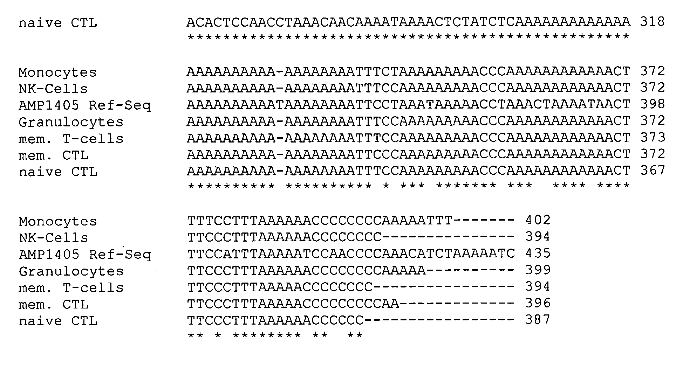

Figure 2 shows the analysis of amplicon No. 1405 (SEQ ID No. 6), bisulfite strand 2, sequencing direction: forward. Relevant positions are indicated in bold.

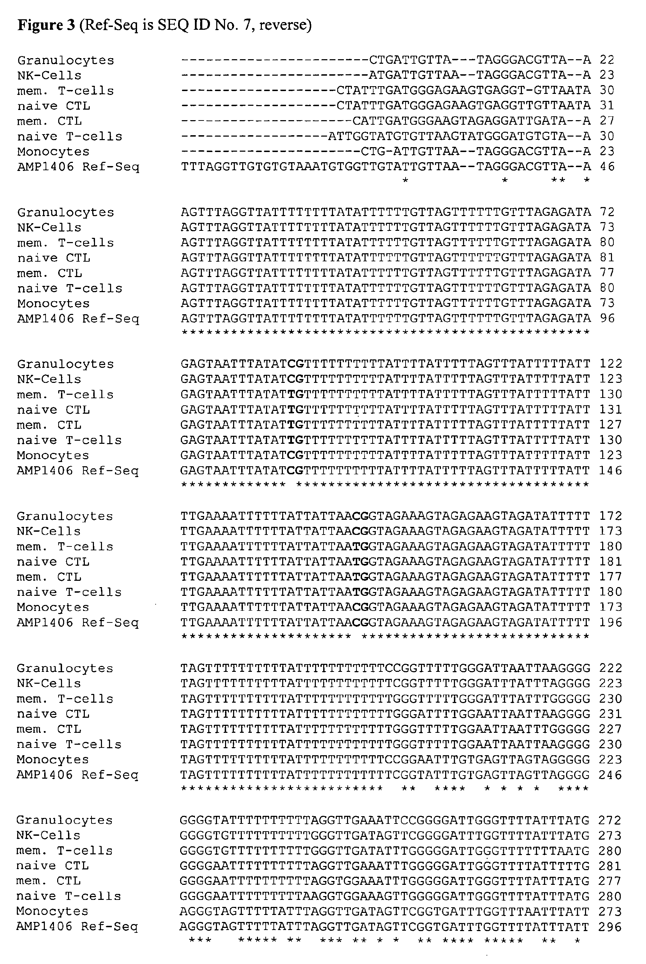

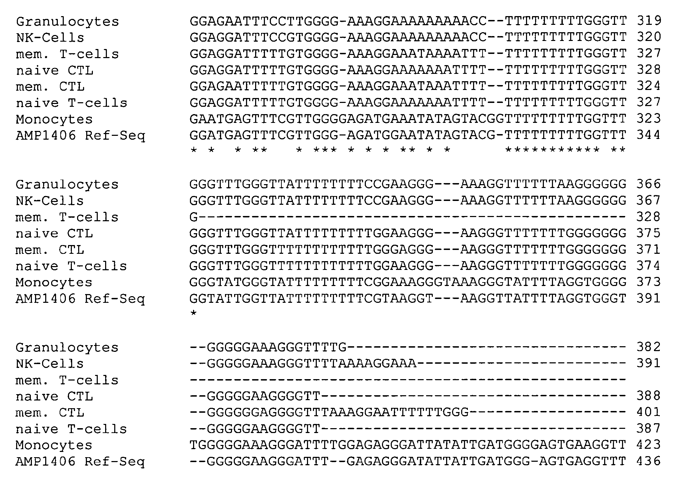

Figure 3 shows the analysis of amplicon No. 1406, bisulfite strand 2, sequencing direction: reverse. Relevant positions are indicated in bold.

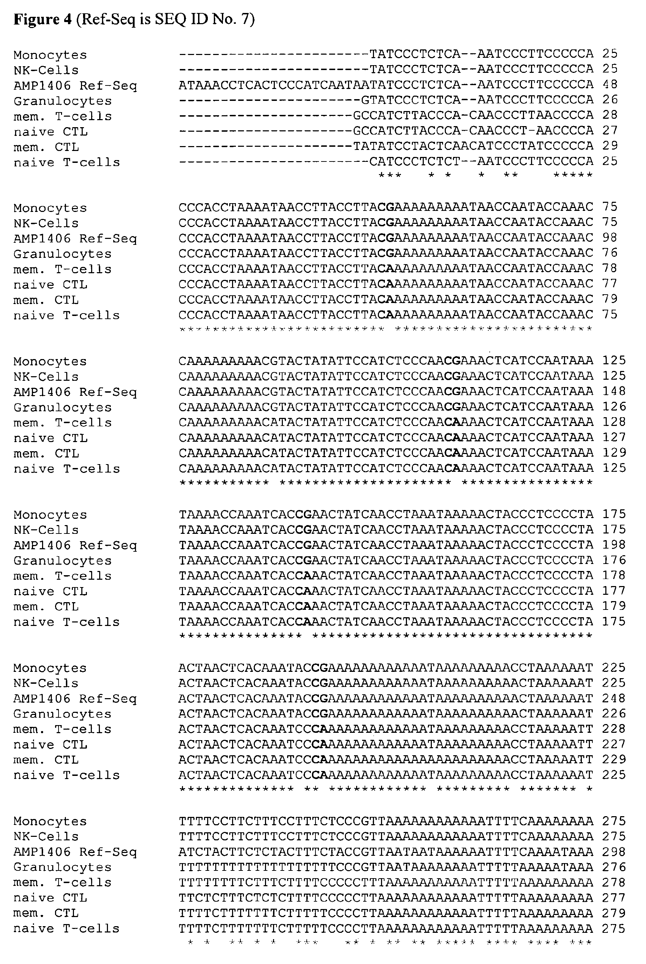

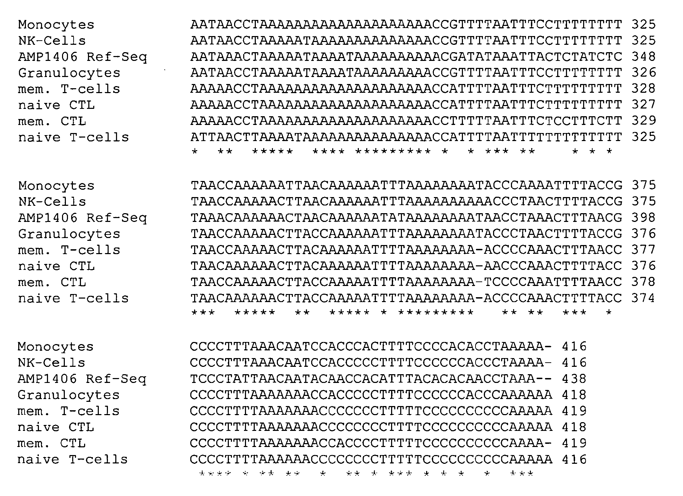

Figure 4 shows the analysis of amplicon No. 1406 (SEQ ID No. 7), bisulfite strand: 2, sequencing direction: forward. Relevant positions are indicated in bold.

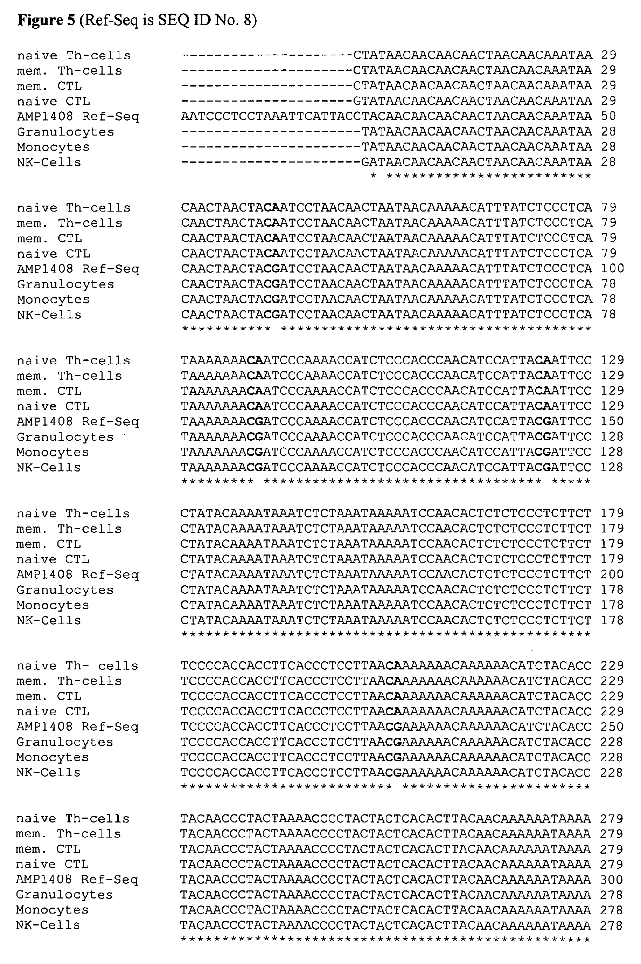

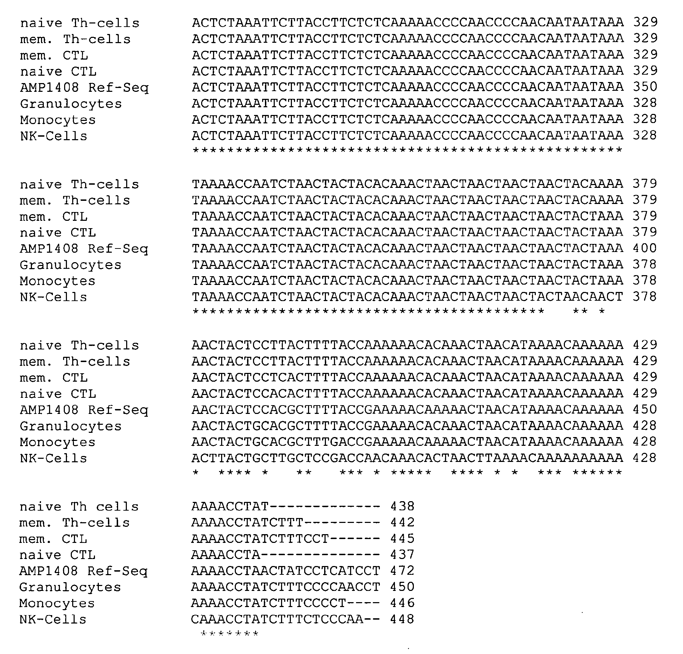

Figure 5 shows the analysis of amplicon No. 1408 (SEQ ID No. 8), bisulfite strand: 2, sequencing direction: forward. Relevant positions are indicated in bold.

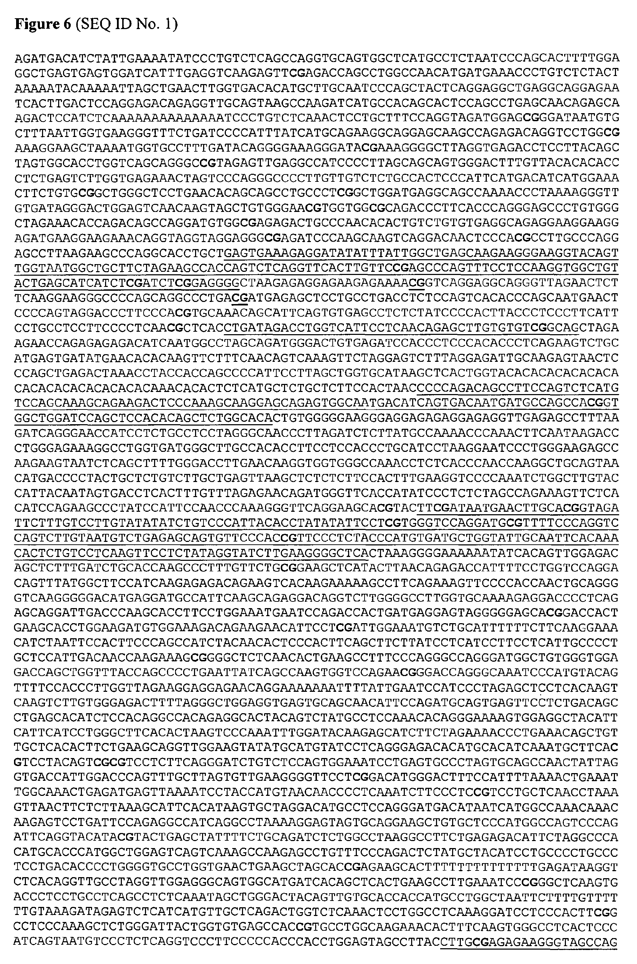

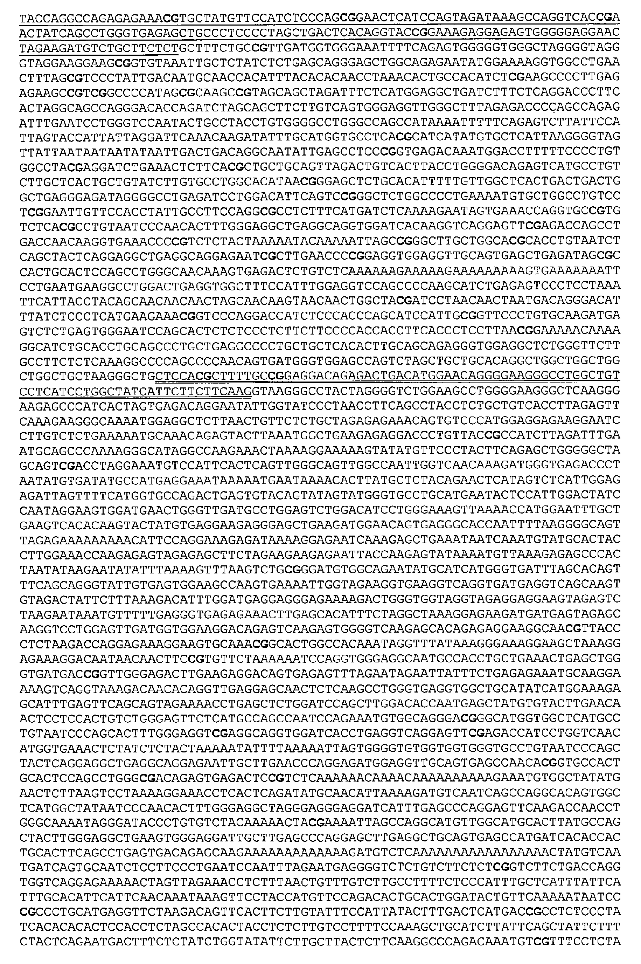

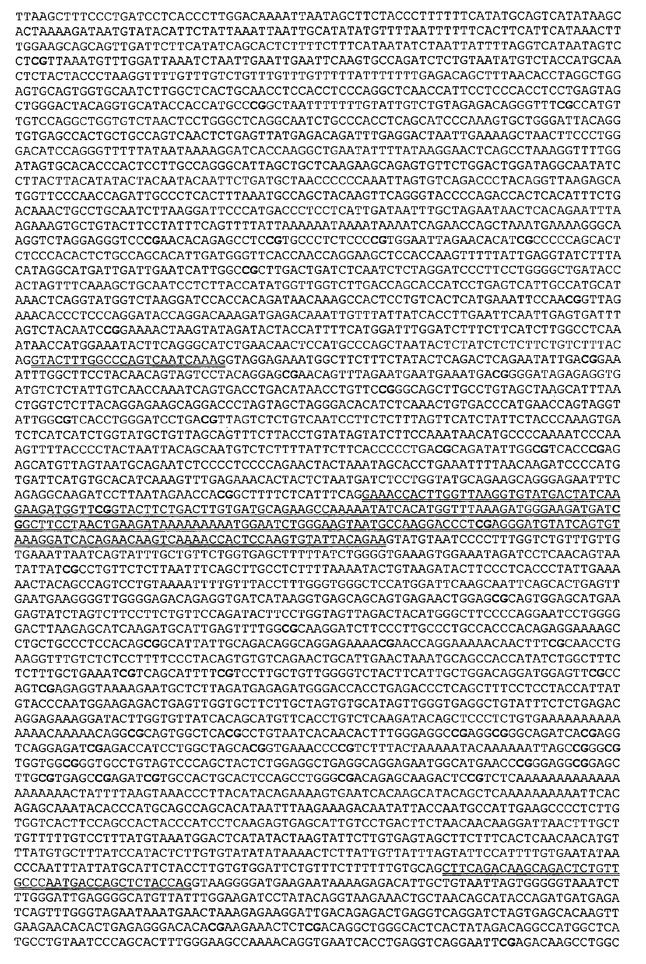

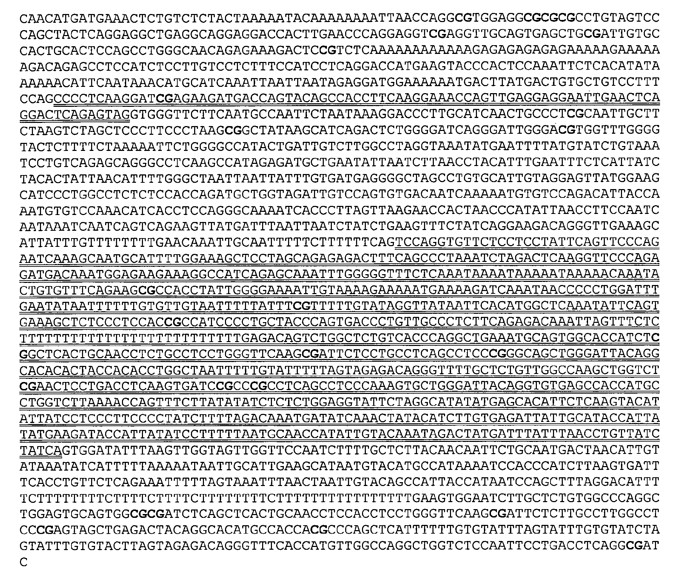

Figure 6 shows the regulatory regions of the γ,δ-T-cell receptor, including regions Nr. 1405 1406 und 1408 on chromosome 11 (NCBI36:11:117714000:117730500:1). Exon sequences are underlined for CD3 δ, and double underlined for CD3 γ. CGs are in bold. SEQ ID No. 1 shows the sequence of the region as considered to have a specific methylation pattern in CD3 cells.
SEQ ID No. 2 to 5 and 9 show the sequences of oligonucleotides used in the Illumina Chip-Fragment assay for CD3γ and CD3δ and CD3ε.
SEQ ID No. 6 to 8 show the reference sequences of amplicon No. 1405 (SEQ ID No. 6), amplicon No. 1406 (SEQ ID No. 7), and amplicon No. 1408 (SEQ ID No. 8).
SEQ ID No. 10 to 38 show the sequences around the CpG positions as analyzed in other preferred T-lymphocyte markers of the present invention according to example 2.

### EXAMPLES

### Example 1 - CD3-Analysis

The inventors have purified various blood subsets, including CD3/CD4, CD3/CD8 naïve and memory T lymphocytes, CD56 natural killer cells, CD19 naïve and memory B cells, CD14 monocytes and CD15 granulocytes. DNA from the purified cells was bisulfite-treated and analysed at various CpG dinucleotide motifs. The inventors then compared the methylation status (finding C as for Cytosine that was methylated in the original sequence versus T for cytosine that was unmethylated in the original sequence).

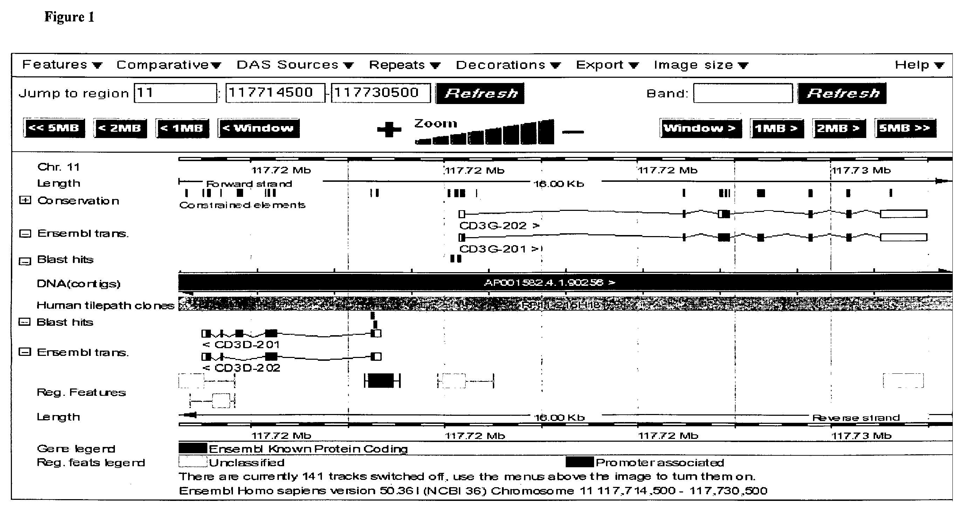

The data showed various CpG motifs and areas in the CD3 γ, δ and ε that were demethylated in all CD3CD4 and CD3CD8 cell types while methylated in all other blood cell types. The differentially methylated gene regions as found for CD3 γ, δ and ε are shown below in Figure 1 and are indicated in bold as "blast hits"

The data, as observed with Illumina Golden Gate technology, show that all CD4 and CD8 positive memory (0.06 and 0.06 respectively) and naïve (0.03 and 0.06, respectively) T cells are subject to much lower methylation rates than all other tested cell types, including CD15 (0.92), CD14 (0.90), CD19 memory and naïve (0.81 and 0.67, respectively), CD56 (0.86) positive blood cells as well as cells derived (0.78) from non-blood tissues. The experimental results are further depicted in the following table.

**Table 1: Methylation analysis results**

| **Gene** | **Chr.** | **S1** n=12; female | **S2** n=5; male | **S3** Pool; female | **S4** Pool; (5 male) | **S5** Pool; (5 male) | **S6** Pool; (5 male) | **S7** Pool; (5 male) | **S8** Pool; (5 male) | **S9** Pool; (5 male) | **S10** Pool; (5 male) | **S11** Pool; (5 male) | **S12** Pool; (5 male) |
|---|---|---|---|---|---|---|---|---|---|---|---|---|---|
| CD3D | 11 | 0.8 | 0.6 | 0.6 | 0.9 | 0.9 | 0.9 | 0.0 | 0.1 | 0.1 | 0.1 | 0.7 | 0.8 |
| | | 0.9 | 0.7 | 0.7 | 0.9 | 0.9 | 0.9 | 0.1 | 0.1 | 0.2 | 0.2 | 0.7 | 0.8 |
| CD3G | 11 | 0.7 | 0.6 | 0.7 | 0.9 | 0.9 | 0.8 | 0.1 | 0.1 | 0.1 | 0.1 | 0.8 | 0.9 |
| | | 0.4 | 0.6 | 0.5 | 0.7 | 0.8 | 0.7 | 0.1 | 0.1 | 0.1 | 0.1 | 0.3 | 0.6 |

| | | | | | | | | | | | | | |
|---|---|---|---|---|---|---|---|---|---|---|---|---|---|
| S1 = ovarian tissue, S2 = whole blood, S3 peripheral blood mononuclear cells, S4 = granulocytes, S5 = monocytes,S6 = NK cells, S7 = naïve T helper cells, S8 = memory T helper cells, S9 = naïve cytotoxic T cells, S10 = memory cytotoxic T cells, S11 = naïve B-cells, S12 = memory B-cells., Chr = chromosome | | | | | | | | | | | | | |

The data showing the high specificity were obtained using a Illumina Golden Gate technology, with the following genomic CpGs regions analysed
**CD3**γ
   cg15880738 (SEQ ID No. 2)
   (+1)AGCTGCTGCACAGGCTGGCTGGCTGGCTGGCTGCTAAGGGCTGCTCCA**CG** cg07545925 (SEQ ID No. 3)
   (+1)**CG**GAAAAACAAAAGGCATCTGCACCTGCAGCCCTGCTGAGGCCCCTGCTG
**CD3δ**
   cg24841244 (SEQ ID No. 4)
   (-1) ACCCAGGCTGATAGTT**CG**GTGACCTGGCTTTATCTACTGGATGAGTTCCG cg07728874 (SEQ ID No. 5)
   (-1) TGGAACATAGCA**CG**TTTCTCTCTGGCCTGGTACTGGCTACCCTTCTCTCG
**CD3ε**
   cg24612198 (Seq ID No. 9)
   AGTCATCTGTTTTGCTTTTTTTCCAGAAGTAGTAAGTCTGCTGGCCTCCG

Since the Illumina technology does only allow the analysis of a single CpG (or rather 2 CpGs per gene locus) the inventors verified the methylation properties using bisulfite sequencing. For that, the inventors bisulfite treated the samples using the Qiagen EpiTect kit, and sequenced the samples using an ABI 3100 prism sequencer. For data interpretation, the KB base calling software supplied by ABI was used.

While not providing entirely quantitative results calculating the percentile of methylated (i.e., CG signal on the plus strand sequence) versus unmethylated (i.e., TG on the plus strand sequence), the data were unambiguous for the purified cell types. All T-lymphocytes were overwhelmingly demethylated at all CG positions analysed, whereas all other analysed cell types were methylated at identical positions.

### Example 2 -Analysis of additional markers in analogy to CD3

In order to identify further suitable markers distingushing and monitoring T-lymphocytes, other markers in addition to CD3 have been identified and tested through methylation analysis. It was found that methylation in the CpG positions in the genes for SLA2, CHRNA3, C16orf24, LCK, FASLG, CD7, SIT1, IL32, CXCR6, UBASH3A, GRAP2, ITGB7 and TXK can also be used in the context of the present invention, as these markers are also able to identify CD3 positive T lymphocytes.

Furthermore, other markers have been identified that identify the subset of the CD8 and CD4 positive cells in the group of CD3 positive T lymphocytes. The genes for GNGT2, CRTAM, IL2RB and ZBTB32 have been found to segregate between CD8 and CD4 positive cells. Equivalently, FLJ00060, FLJ38379, PPP6C, CD226, ZBTB7B and TNFAIP8 are capable of positively identifying CD4 expressing cells in whole blood and segregate between CD4 and CD8 positive CD3 positive cells.

The following table 2 summarizes the Illumina-data as obtained for the above markers at selected CpG positions. It can be seen that several other markers can be used in order to selectively identify CD4+ (for CD3+ lymphocyte subset-identification), namely FLJ00060; FLJ38379; PPP6C; CD226; ZBTB7B and/or TNFAIP8, in order to selectively identify CD8+ (for CD3+ lymphocyte subset-identification), namely GNGT2; CRTAM; IL2RB and/or ZBTB32, and in order to selectively identify CD3+ lymphocytes, namely CD3D; CD3G, and/or CD3E, and/or SLA2,CHRNA3, C16orf24; LCK; FASLG; FASLG; CD7; SIT1; IL32; CXCR6; UBASH3A; GRAP2; ITGB7 and/or TXK, as shown with selected CpG sites as preferred examples. Based on the table, the person of skill will be able to extend the teaching regarding CD3 as herein to these markers and their CpG sites.

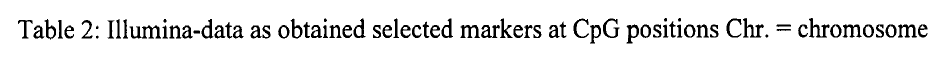

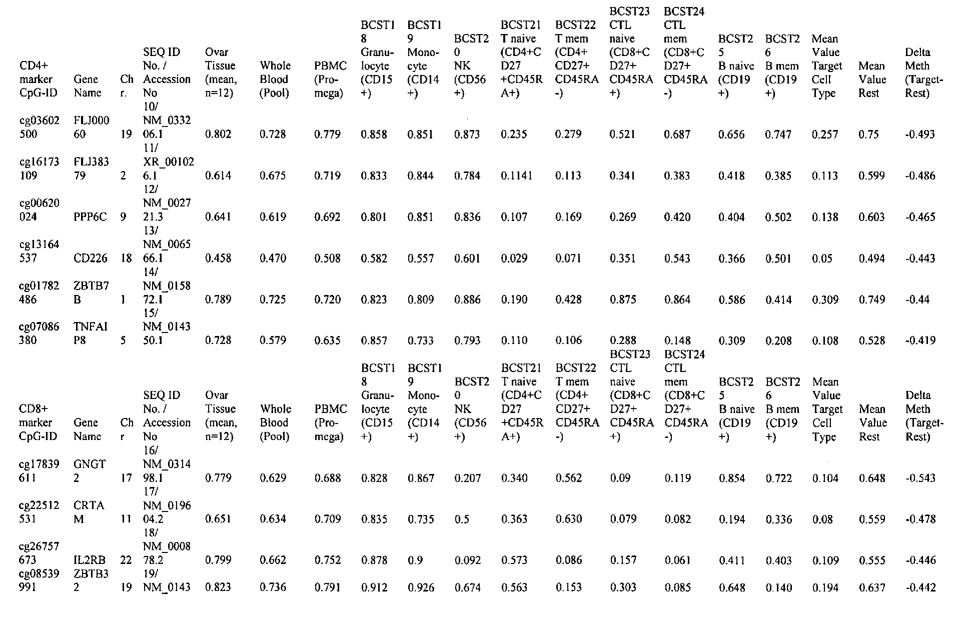

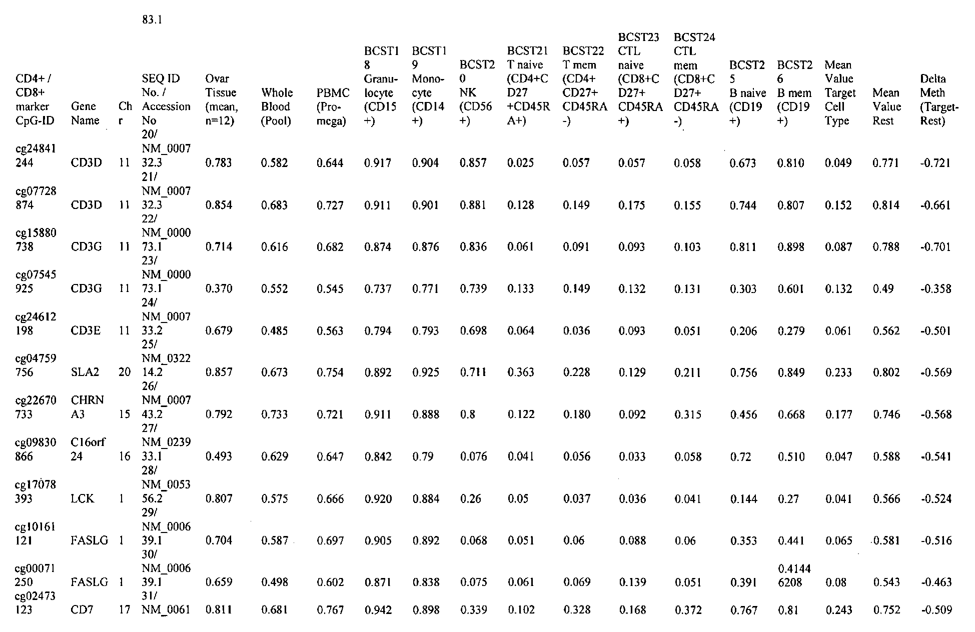

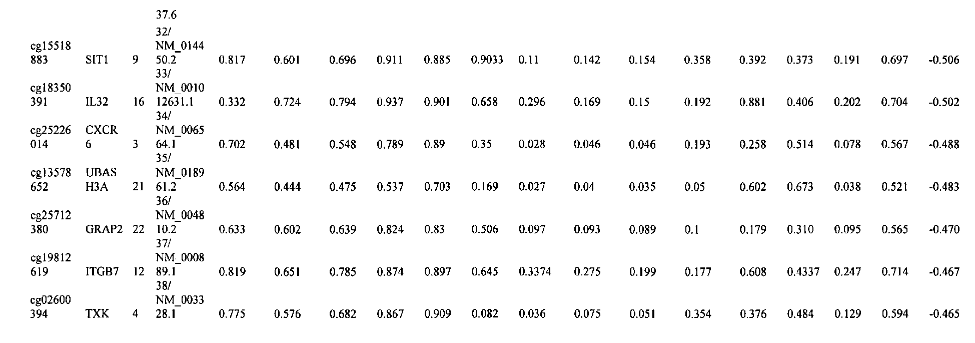

## Claims

1. A method for identifying CD3⁺ CD4⁺, and/or CD3⁺ CD8⁺ naïve and/or memory T-lymphocytes in a sample derived from a mammal comprising T cells, comprising
a) analyzing the methylation status of at least one CpG position in the T-lymphocyte specific demethylated region located in amplicon 1405 (SEQ ID No. 6), amplicon 1406 (SEQ ID No. 7), and/or amplicon 1408 (SEQ ID No. 8), and
b) identifying CD3⁺ CD4⁺, and/or CD3⁺ CD8⁺ naive and/or memory T-lymphocytes based on said methylation status, wherein a demethylation of at least one CpG position in said amplicon to at least 90% is indicative for a CD3⁺ CD4⁺, and/or CD3⁺ CD8⁺ naive and/or memory T-lymphocyte cell.

2. The method according to claim 1, wherein said at least one CpG position in said sample is demethylated to more than 91 % and preferably more than 92% and most preferred more than 95%.

3. The method according to claim 1 or 2, wherein the analysis of the methylation status comprises a method selected from methylation specific enzymatic digests, bisulphite sequencing, analysis selected from promoter methylation, CpG island methylation, MSP, HeavyMethyl, MethyLight, Ms-SNuPE or other methods relying on a detection of amplified DNA.

4. The method according to any of claims 1 to 3, further comprising a methylation analysis of at least one CpG position in the genes for CD4⁺ and/or CD8, in particular CD8 beta, or in the genes for GNGT2, CRTAM, IL2RB, and ZBTB32, or FLJ00060, FLJ38379, PPP6C, CD226, ZBTB7B, and TNFAIP8, or in the genes SLA2, CHRNA3, C16orf24, LCK, FASLG, CD7, SIT1, IL32, CXCR6, UBASH3A, GRAP2, ITGB7 or TXK.

5. The method according to any of claims 1 to 4, wherein said method is suitable for routine application, for example on a DNA-chip.

6. The method according to any of claims 1 to 5, wherein said identification comprises a distinction of said T-lymphocytes from all major peripheral blood cell types or non-blood cells.

7. The method according to any of claims 1 to 6, wherein said sample is selected from a human body fluid, including human blood samples, or a tissue, organ or cell type blood sample, a sample of blood lymphocytes or a fraction thereof.

8. The method according to any of claims 1 to 7, further comprising the step of concluding on the immune status of said human based on said T-lymphocytes as identified.

9. The method according to any of claims 1 to 8, wherein said human suffers from or is likely to suffer from autoimmune diseases, transplant rejections, cancer, and/or allergy.

10. Use of a kit comprising materials for performing the method according to claim 1 for identifying CD3⁺ CD4⁺, and/or CD3⁺ CD8⁺ naive and/or memory T-lymphocytes in a sample, said kit comprising:
a) a bisulfite reagent, and
b) materials for performing a methylation analysis for CpG positions in the TLSDR-region selected from CpG position 1, 2, 3, 4, 5, 6, 7, 8, 9, and 10 as numerically counted from the 5'- end of the sequence located in amplicon 1405 (SEQ ID No. 6), CpG position 1, 2, 3, 4, 5, 6, 7, and 8 of the sequence located in amplicon 1406 (SEQ ID No. 7), and CpG position 1, 2, 3, 4, 5, 6, 7 of the sequence located in amplicon 1408 (SEQ ID No. 8 for identifying CD3⁺ CD4⁺, and/or CD3⁺ CD8⁺ naïve and/or memory T-lymphocytes in a mammal.

## Patentansprüche

1. Verfahren zur Identifizierung von CD3⁺ CD4⁺ und/oder CD3⁺ CD8⁺ naïven und/oder memory T-Lymphocyten in einer von einem Säuger abgeleiteten Probe, die Zellen umfasst, umfassend
a) Analysieren des Methylierungsstatus von mindestens einer CpG Position in der T-Lymphocyten spezifischen demethylierten Region lokalisiert in Amplikon 1405 (SEQ ID No. 6), Amplikon 1406 (SEQ ID No. 7) und/oder Amplikon 1408 (SEQ ID No. 8), und
b) Identifizierung von CD3⁺ CD4⁺ und/oder CD3⁺ CD8⁺ naïven und/oder memory T-Lymphocyten basierend auf dem Methylierungsstatus, wobei eine Demethylierung von mindestens einer CpG Position in dem Amplikon zu mindestens 90% eine CD3⁺ CD4⁺ und/oder CD3⁺ CD8⁺ naïve und/oder memory T-Lymphocytenzelle anzeigt.

2. Verfahren nach Anspruch 1, wobei die mindestens eine CpG Position in der Probe zu mehr als 91% und bevorzugt zu mehr als 92% und am meisten bevorzugt zu mehr als 95% demethyliert ist.

3. Verfahren nach Anspruch 1 oder 2, wobei die Analyse des Methylierungsstatus ein Verfahren ausgewählt aus Methylierungs-spezifischen enzymatischen Verdaus, Bisulfit-Sequenzierung, eine Analyse ausgewählt aus Promoter-Methylierung, CpG Island-Methylierung, MSP, HeavyMethyl, MethyLight, Ms-SNuPE oder anderen Verfahren umfasst, die auf einem Nachweis von amplifizierter DNA beruhen.

4. Verfahren nach einem der Ansprüche 1 bis 3, weiter umfassend eine Methylierungsanalyse von mindestens einer CpG Position in den Genen für CD4⁺ und/oder CD8, insbesondere CD8 beta, oder in den Genen für GNGT2, CRTAM, IL2RB und ZBTB32, oder FLJ00060, FLJ38379, PPP6C, CD226, ZBTB7B und TNFAIP8, oder in den Genen SLA2, CHRNA3, C16orf24, LCK, FASLG, CD7, SIT1, IL32, CXCR6, UBASH3A, GRAP2, ITGB7 oder TXK.

5. Verfahren nach einem der Ansprüche 1 bis 4, wobei das Verfahren für die Routineanwendung geeignet ist, zum Beispiel auf einem DNA-Chip.

6. Verfahren nach einem der Ansprüche 1 bis 5, wobei die Identifizierung comprises a distinction of said T-lymphocytes from all major peripheral blood cell types or non-blood cells umfasst.

7. Verfahren nach einem der Ansprüche 1 bis 6, wobei die Probe ausgewählt ist aus einer menschlichen Körperflüssigkeit, einschließlich menschlichen Blutproben oder einem Gewebe, Organ oder einer Zelltyp-Blutprobe, einer Probe von Blut-Lymphocyten oder einer Fraktion davon.

8. Verfahren nach einem der Ansprüche 1 bis 7, weiter umfassend den Schritt von Schlussfolgern auf den Immunstatus des Menschen, basierend auf den T-Lymphocyten wie identifiziert.

9. Verfahren nach einem der Ansprüche 1 bis 8, wobei der Mensch an einer Autoimmunerkrankung, Transplantabstossungen, Krebs und/oder Allergie leidet, oder wahrscheinlich daran leiden wird.

10. Verwendung eines Kits umfassend Materialien zur Durchführung des Verfahrens nach Anspruch 1 zur Identifizierung von CD3⁺ CD4⁺ und/oder CD3⁺ CD8⁺ naïven und/oder memory T-Lymphocyten in einer Probe, wobei das Kit umfasst:
a) ein Bisulfitreagenz, und
b) Materialien zur Durchführung einer Methylierungsanalyse für CpG Positionen in der TLSDR-Region ausgewählt aus CpG Position 1, 2, 3, 4, 5, 6, 7, 8, 9 und 10 wie numerisch vom 5 '-Ende der Sequenz lokalisert in Amplikon 1405 (SEQ ID No. 6) gezählt, CpG Position 1, 2, 3, 4, 5, 6, 7 und 8 der Sequenz lokalisert in Amplikon 1406 (SEQ ID No. 7) und CpG Position 1, 2, 3, 4, 5, 6, 7 der Sequenz lokalisert in Amplikon 1408 (SEQ ID No. 8) zur Identifizierung von CD3⁺ CD4⁺ und/oder CD3⁺ CD8⁺ naïven und/oder memory T-Lymphocyten in einem Säuger.

## Revendications

1. Procédé pour identifier des lymphocytes T naïfs et/ou à mémoire CD3⁺ CD4⁺, et/ou CD3⁺ CD8⁺ dans un échantillon issu d'un mammifère, comprenant des cellules T, comprenant le fait
a) d'analyser l'état de méthylation d'au moins une position CpG dans la région déméthylée spécifique des lymphocytes T située dans l'amplicon 1405 (SEQ ID N° 6), l'amplicon 1406 (SEQ ID n° 7), et/ou l'amplicon 1408 (SEQ ID N° 8), et
b) d'identifier des lymphocytes T naïfs et/ou à mémoire CD3⁺ CD4⁺, et/ou CD3⁺ CD8⁺ sur la base dudit état de méthylation, où une déméthylation d'au moins une position CpG dans ledit amplicon à au moins 90% indique une cellule lymphocytaire T naïve et/ou à mémoire CD3⁺ CD4⁺, et/ou CD3⁺ CD8⁺.

2. Procédé selon la revendication 1, dans lequel ladite au moins une position CpG dans ledit échantillon est déméthylée à plus de 91% et de préférence à plus de 92% et plus préférablement à plus de 95%.

3. Procédé selon la revendication 1 ou 2, dans lequel l'analyse de l'état de méthylation comprend un procédé choisi parmi une digestion enzymatique spécifique de la méthylation, un séquençage au bisulfite, une analyse choisie parmi la méthylation du promoteur, la méthylation d'îlots CpG, MSP, HeavyMethyl, MethyLight, Ms-SNuPE ou d'autres procédés dépendant d'une détection d'ADN amplifié.

4. Procédé selon l'une des revendications 1 à 3, comprenant en outre une analyse de méthylation d'au moins une position CpG dans les gènes pour CD4⁺ et/ou CD8, en particulier CD8 bêta, ou dans les gènes pour GNGT2, CRTAM, IL2RB, et ZBTB32 ou FLJ00060, FLJ38379, PPP6C, CD226, ZBTB7B, et TNFAIP8, ou dans les gènes SLA2, CHRNA3, C16orf24, LCK, FASLG, CD7, SIT1, IL32, CXCR6, UBASH3A, GRAP2, ITGB7 ou TXK.

5. Procédé selon l'une des revendications 1 à 4, dans lequel ledit procédé est approprié pour une application de routine, par exemple sur une puce à ADN.

6. Procédé selon l'une des revendications 1 à 5, dans lequel ladite identification comprend une distinction desdits lymphocytes T de tous les principaux types de cellules sanguines périphériques ou de cellules non-sanguines.

7. Procédé selon l'une des revendications 1 à 6, dans lequel ledit échantillon est choisi parmi un fluide corporel humain, y compris des échantillons de sang humain ou un échantillon de tissu, d'organe ou de sang type cellule, un échantillon de lymphocytes de sang ou une fraction de ceux-ci.

8. Procédé selon l'une des revendications 1 à 7, comprenant en outre l'étape qui consiste à conclure sur l'état immunitaire dudit être humain sur la base desdits lymphocytes T tels qu'identifiés.

9. Procédé selon l'une des revendications 1 à 8, dans lequel ledit être humain souffre ou risque de souffrir de maladies auto-immunes, de rejets de greffe, de cancer, et/ou d'allergie.

10. Utilisation d'un kit comprenant des matériaux pour réaliser le procédé selon la revendication 1 afin d'identifier des lymphocytes T naïfs et/ou à mémoire CD3⁺ CD4⁺, et/ou CD3⁺ CD8⁺ dans un échantillon, ledit kit comprenant :
a) un réactif bisulfite, et
b) des matériaux pour réaliser une analyse de méthylation pour des positions CpG dans la région TLSDR choisies parmi la position CpG 1, 2, 3, 4, 5, 6, 7, 8, 9, et 10 telles que comptées numériquement à partir de l'extrémité 5' de la séquence située dans l'amplicon 1405 (SEQ ID N° 6), la position CpG 1, 2, 3, 4, 5, 6, 7, et 8 de la séquence située dans l'amplicon 1406 (SEQ ID N° 7), et la position CpG 1, 2, 3, 4, 5, 6, 7 de la séquence située dans l'amplicon 1408 (SEQ ID N° 8) pour identifier des lymphocytes T naïfs et/ou à mémoire CD3⁺ CD4⁺, et/ou CD3⁺ CD8⁺ chez un mammifère.


### SEQUENCE LISTING

<110> Epiontis GmbH
<120> Epigenetic markers for the identification of blood sub-cells of type 1
<130> FB20882
<160> 38
<170> PatentIn version 3.4
<210> 1
   <211> 16501
   <212> DNA
   <213> Homo sapiens
<400> 1
   agatgacatc tattgaaaat atccctgtct cagccaggtg cagtggctca tgcctctaat     60
   cccagcactt ttggaggctg agtgagtgga tcatttgagg tcaagagttc gagaccagcc    120
   tggccaacat gatgaaaccc tgtctctact aaaaatacaa aaattagctg aacttggtga    180
   cacatgcttg caatcccagc tactcaggag gctgaggcag gagaatcact tgactccagg    240
   agacagaggt tgcagtaagc caagatcatg ccacagcact ccagcctgag caacagagca    300
   agactccatc tcaaaaaaaa aaaaaatccc tgtctcaaac tcctgctttc caggtagatg    360
   gagcgggata atgtgcttta attggtgaag ggtttctgat ccccatttat catgcagaag    420
   gcaggagcaa gccagagaca ggtcctggcg aaaggaagct aaaatggtgc ctttgataca    480
   ggggaaaggg atacgaaagg ggcttaggtg agacctcctt acagctagtg gcacctggtc    540
   agcagggccg tagagttgag gccatcccct tagcagcagt gggactttgt tacacacacc    600
   ctctgagtct tggtgagaaa ctagtcccag ggccccttgt tgtctctgcc actcccattc    660
   atgacatcat ggaaacttct gtgcggctgg gctcctgaac acagcagcct gccctcggct    720
   ggatgaggca gccaaaaccc taaaagggtt gtgataggga ctggagtcaa caagtagctg    780
   tgggaacgtg gtggcgcaga cccttcaccc agggagccct gtgggctaga aacaccagac    840
   agccaggatg tggcgagaga ctgcccaaca cactgtctgt gtgaggcaga ggaaggaagg    900
   agatgaagga agaaacaggt aggtaggagg gcgagatccc aagcaagtca ggacaactcc    960
   cacgccttgc ccaggagcct taagaagccc aggcacctgc tgagtgaaag aggatatatt   1020
   tattggctga gcaagaaggg aaggtacagt tggtaatggc tgcttctaga agccaccagt   1080
   ctcaggttca cttgttccga gcccagtttc ctccaaggtg gctgtactga gcatcatctc   1140
   gatctcggag gggctaagag aggagaagag aaaacggtca ggaggcaggg ttagaactct   1200
   tcaaggaagg gccccagcag gccctgacga tgagagctcc tgcctgacct ctccagtcac   1260
   acccagcaat gaactcccca gtaggaccct tcccacgtgc aaacagcatt cagtgtgagc   1320
   ctctctatcc ccacttaccc tcccttcatt cctgcctcct tcccctcaac gctcacctga   1380
   tagacctggt cattcctcaa cagagcttgt gtgtcggcag ctagaagaac cagagagaga   1440
   catcaatggc ctagcagatg ggactgtgag atccaccctc ccacaccctc agaagtctgc   1500
   atgagtgata tgaacacaca agttctttca acagtcaaag ttctaggagt ctttaggaga   1560
   ttgcaagagt aactcccagc tgagactaaa cctaccacca gccccattcc ttagctggtg   1620
   cataagctca ctggtacaca cacacacaca cacacacaca cacacacaaa cacactctca   1680
   tgctctgctc ttccactaac ccccagacag ccttccagtc tcatgtccag caaagcagaa   1740
   gactcccaaa gcaaggagca gagtggcaat gacatcagtg acaatgatgc cagccacggt   1800
   ggctggatcc agctccacac agctctggca cactgtgggg gaagggagga gagaggagag   1860
   gttgagagcc tttaagatca gggaaccatc ctctgcctcc tagggcaacc cttagatctc   1920
   ttatgccaaa acccaaactt caataagacc ctgggagaaa ggcctggtga tgggcttgcc   1980
   acaccttcct ccaccctgca tcctaaggaa tccctgggaa gagccaagaa gtaatctcag   2040
   cttttgggac cttgaacaag gtggtgggcc aaacctctca cccaaccaag gctgcagtaa   2100
   catgacccct actgctctgt cttgctgagt taagctctct cttccacttt gaaggtcccc   2160
   aaatctggct tgtaccatta caatagtgac ctcactttgt ttagagaaca gatgggttca   2220
   ccatatccct ctctagccag aaagttctca catccagaag ccctatccat tccaacccaa   2280
   agggttcagg aagcacgtac ttcgataatg aacttgcacg gtagattctt tgtccttgta   2340
   tatatctgtc ccattacacc tatatattcc tcgtgggtcc aggatgcgtt ttcccaggtc   2400
   cagtcttgta atgtctgaga gcagtgttcc caccgttccc tctacccatg tgatgctggt   2460
   attgcaattc acaaacactc tgtcctcaag ttcctctata ggtatcttga aggggctcac   2520
   taaaggggaa aaaatatcac agttggagac agctctttga tctgcaccaa gccctttgtt   2580
   ctgcggaagc tcatacttaa cagagaccat tttcctggtc caggacagtt tatggcttcc   2640
   atcaagagag acagaagtca caagaaaaag ccttcagaaa gttccccacc aactgcaggg   2700
   gtcaaggggg acatgaggat gccattcaag cagaggacag gtcttggggc cttggtgcaa   2760
   aagaggaccc ctcagagcag gattgaccca agcaccttcc tggaaatgaa tccagaccac   2820
   tgatgaggag tagggggagc acggaccact gaagcacctg gaagatgtgg aaagacagaa   2880
   gaacattcct cgattggaaa tgtctgcatt ttttcttcaa ggaaacatct aattccactt   2940
   cccagccatc tacaacactc ccacttcagc ttcttatcct catccttcct cattgcccct   3000
   gctccattga caaccaagaa agcggggctc tcaacactga agcctttccc agggccaggg   3060
   atggctgtgg gtggagacca gctggtttac cagcccctga attatcagcc aagtggtcca   3120
   gaacgggacc agggcaaatc ccatgtacag ttttccaccc ttggttagaa ggaggagaac   3180
   aggaaaaaaa ttttattgaa tccatcccta gagctcctca caagtcaagt cttgtgggag   3240
   acttttaggg ctggaggtga gtgcagcaac attccagatg cagtgagttc ctctgacagc 3300
   ctgagcacat ctccacaggc cacagaggca ctacagtcta tgcctccaaa cacagggaaa 3360
   agtggaggct acattcattc atcctgggct tcacactaag tcccaaattt ggatacaaga 3420
   gcatcttcta gaaaaccctg aaacagctgt tgctcacact tctgaagcag gttggaagta 3480
   tatgcatgta tcctcaggga gacacatgca catcaaatgc ttcacgtcct acagtcgcgt 3540
   cctcttcagg gatctgtctc cagtggaaat cctgagtgcc ctagtgcagc caactattag 3600
   gtgaccattg gacccagttt gcttagtgtt gaaggggttc ctcggacatg ggactttcca 3660
   ttttaaaact gaaattggca aactgagatg agttaaaatc ctaccatgta acaacccctc 3720
   aaatcttccc tccgtcctgc tcaacctaaa gttaacttct cttaaagcat tcacataagt 3780
   gctaggacat gcctccaggg atgacataat catggccaaa caaacaagag tcctgattcc 3840
   agaggccatc aggcctaaaa ggagtagtgc aggaagctgt gctcccatgg ccagtcccag 3900
   attcaggtac atacgtactg agctattttc tgcagatctc tggcctaagg ccttctgaga 3960
   gacattctag gcccacatgc acccatggct ggagtcagtc aaagccaaga gcctgtttcc 4020
   cagactctat gctacatcct gcccctgccc tcctgacacc cctggggtgc ctggtgaact 4080
   gaagctagca ccgagaagca cttttttttt tttttgagat aaggtctcac aggttgccta 4140
   ggttggaggg cagtggcatg atcacagctc actgaagcct tgaaatcccg ggctcaagtg 4200
   accctcctgc ctcagcctct caaatagctg ggactacagt tgtgcaccac catgcctggc 4260
   taattctttt gttttttgta aagatagagt ctcatcatgt tgctcagact ggtctcaaac 4320
   tcctggcctc aaaggatcct cccacttcgg cctcccaaag ctctgggatt actggtgtga 4380
   gccaccgtgc ctggcaagaa acactttcaa gtgggcctca ctcccatcag taatgtccct 4440
   ctcaggtccc ttcccccacc cacctggagt agccttacct tgcgagagaa gggtagccag 4500
   taccaggcca gagagaaacg tgctatgttc catctcccag cggaactcat ccagtagata 4560
   aagccaggtc accgaactat cagcctgggt gagagctgcc ctcccctagc tgactcacag 4620
   gtaccggaaa gaggagagtg ggggaggaac tagaagatgt ctgcttctct gctttctgcc 4680
   gttgatggtg ggaaattttc agagtggggg tgggctaggg gtagggtagg aaggaagcgg 4740
   tgtaaattgc tctatctctg agcagggagc tggcagagaa tatggaaaag gtggcctgaa 4800
   ctttagcgtc cctattgaca atgcaaccac atttacacac aacctaaaca ctgccacatc 4860
   tcgaagcccc ttgagagaag ccgtcggccc catagcgcaa gccgtagcag ctagatttct 4920
   catggaggct gatctttctc aggacccttc actaggcagc cagggacacc agatctagca 4980
   gcttcttgtc agtgggaggt tgggctttag agaccccagc cagagatttg aatcctgggt 5040
   ccaatactgc ctacctgtgg ggcctgggcc agccataaaa tttttcagag tcttattcca 5100
   ttagtaccat tattaggatt caaacaagat atttgcatgg tgcctcacgc atcatatgtg 5160
   ctcattaagg ggtagttatt aataataata taattgactg acaggcaata ttgagcctcc 5220
   cggtgagaca aatggacctt tttcccctgt ggcctacgag gatctgaaac tcttcacgct 5280
   gctgcagtta gactgtcact tacctgggga cagagtcatg cctgtcttgc tcactgctgt 5340
   atcttgtgcc tggcacataa cgggagctct gcacattttt gttggctcac tgactgactg 5400
   gctgagggag ataggggcct gagatcctgg acattcagtc cgggctctgg cccctgaaaa 5460
   tgtgctggcc tgtcctcgga attgttccac ctattgcctt ccaggcgcct ctttcatgat 5520
   ctcaaaagaa tagtgaaacc aggtgccgtg tctcacgcct gtaatcccaa cactttggga 5580
   ggctgaggca ggtggatcac aaggtcagga gttcgagacc agcctgacca acaaggtgaa 5640
   accccgtctc tactaaaaat acaaaaatta gccgggcttg ctggcacgca cctgtaatct 5700
   cagctactca ggaggctgag gcaggagaat cgcttgaacc ccggaggtgg aggttgcagt 5760
   gagctgagat agcgccactg cactccagcc tgggcaacaa agtgagactc tgtctcaaaa 5820
   aagaaaaaga aaaaaaaagt gaaaaaaatt cctgaatgaa ggcctggact gaggtggctt 5880
   tccatttgga ggtccagccc caagcatctg agagtccctc ctaaattcat tacctacagc 5940
   aacaacaact agcaacaagt aacaactggc tacgatccta acaactaatg acagggacat 6000
   ttatctccct catgaagaaa cggtcccagg accatctccc acccagcatc cattgcggtt 6060
   ccctgtgcaa gatgagtctc tgagtgggaa tccagcactc tctccctctt cttccccacc 6120
   accttcaccc tccttaacgg aaaaacaaaa ggcatctgca cctgcagccc tgctgaggcc 6180
   cctgctgctc acacttgcag cagagggtgg aggctctggg ttcttgcctt ctctcaaagg 6240
   ccccagcccc aacagtgatg ggtggagcca gtctagctgc tgcacaggct ggctggctgg 6300
   ctggctgcta agggctgctc cacgcttttg ccggaggaca gagactgaca tggaacaggg 6360
   gaagggcctg gctgtcctca tcctggctat cattcttctt caaggtaagg gcctactagg 6420
   ggtctggaag cctggggaag ggctcaaggg aagagcccat cactagtgag acaggaatat 6480
   tggtatccct aaccttcagc ctacctctgc tgtcacctta gagttcaaag aagggcaaaa 6540
   tggaggctct taactgttct ctgctagaga gaaacagtgt cccatggagg agaaggaatc 6600
   cttgtctctg aaaaatgcaa acagagtact taaatggctg aagagaggac cctgttaccg 6660
   ccatcttaga tttgaatgca gcccaaaagg gcataggcca agaaactaaa aggaaaaagt 6720
   atatgttccc tacttcagag ctgggggcta gcagtcgacc taggaaatgt ccattcactc 6780
   agttgggcag ttggccaatt ggtcaacaaa gatgggtgag accctaatat gtgatatgcc 6840
   atgaggaaat aaaaatgaat aaaacactta tgctctacag aactcatagt ctcattggag 6900
   agattagttt tcatggtgcc agactgagtg tacagtatag tatgggtgcc tgcatgaata 6960
   ctccattgga ctatccaata ggaagtggat gaactgggtt gatgcctgga gtctggacat 7020
   cctgggaaag ttaaaaccat ggaatttgct gaagtcacac aagtactatg tgaggaagag   7080
   ggagctgaag atggaacagt gagggcacca attttaaggg gcagttagag aaaaaaaaac   7140
   attccaggaa agagataaaa ggagaatcaa agagctgaaa taatcaaatg tatgcactac   7200
   cttggaaacc aagagagtag agagcttcta gaagaagaga attaccaaga gtataaaatg   7260
   ttaaagagag cccactaata taagaatata tttaaaagtt taagtctgcg ggatgtggca   7320
   gaatatgcat catgggtgat ttagcacagt ttcagcaggg tattgtgagt ggaagccaag   7380
   tgaaaattgg tagaaggtga aggtcaggtg atgaggtcag caagtgtaga ctattcttta   7440
   aagacatttg gatgaggagg gagaaaagac tgggtggtag gtagaggagg aagtagagtc   7500
   taagaataaa tgtttttgag ggtgagagaa acttgagcac atttctaggc taaaggagaa   7560
   gatgatgagt agagcaaggt cctggagttg atggtggaag gacagagtca agagtggggt   7620
   caagagcaca gagaggaagg caacgttacc ctctaagacc aggagaaagg aagtgcaaac   7680
   ggcactggcc acaaataggt ttataaaggg aaaggaagct aaaggagaaa ggacaataac   7740
   aacttccgtg ttctaaaaaa tccaggtggg aggcaatgcc acctgctgaa actgagctgg   7800
   gtgatgaccg gttgggagac ttgaagagga cagtgagagt ttagaataga attatttctg   7860
   agagaaatgc aaggaaaagt caggtaaaga caacacaggt tgaggagcaa ctctcaagcc   7920
   tgggtgaggt ggctgcatat catggaaaga gcatttgagt tcagcagtag aaaacctgag   7980
   ctctggatcc agcttggaca ccaatgagct atgtgtactt gaacaactcc tccactgtct   8040
   gggagttctc atgccagcca atccagaaat gtggcaggga cgggcatggt ggctcatgcc   8100
   tgtaatccca gcactttggg aggtcgaggc aggtggatca cctgaggtca ggagttcgag   8160
   accatcctgg tcaacatggt gaaactctat ctctactaaa aatattttaa aaattagtgg   8220
   ggtgtggtgg tgggtgcctg taatcccagc tactcaggag gctgaggcag gagaattgct   8280
   tgaacccagg agatggaggt tgcagtgagc caacacggtg ccactgcact ccagcctggg   8340
   cgacagagtg agactccgtc tcaaaaaaca aaacaaaaaa aaaagaaatg tggctatatg   8400
   aactcttaag tcctaaaagg aaacctcact cagatatgca acattaaaag atgtcaatca   8460
   gccaggcaca gtggctcatg gctataatcc caacactttg ggaggctagg gagggaggat   8520
   catttgagcc caggagttca agaccaacct gggcaaaata gggataccct gtgtctacaa   8580
   aaactacgaa aattagccag gcatgttggc atgcacttat gccagctact tgggaggctg   8640
   aagtgggagg attgcttgag cccaggagct tgaggctgca gtgagccatg atcacaccac   8700
   tgcacttcag cctgagtgac agagcaagaa aaaaaaaaaa agatgtctca aaaaaaaaaa   8760
   aaaaaactat gtcaatgatc agtgcaatct ccttccctga atccaattta gaatgagggg   8820
   tctctgtctt ctctcggtct tctgaccagg tggtcaggag aaaaactagt tagaaacctc   8880
   tttaactgtt tgtcttgcct tttctcccat ttgctcattt attcatttgc acattcattc 8940
   aacaaataaa gttcctacca tgttccagac actgcactgg atactgttca aaaataatcc 9000
   cgccctgcat gaggttctaa gacagttcac ttcttgtatt tccattatac tttgactcat 9060
   gaccgcctct ccctatcaca cacactccac ctctagccac actacctctc ttgtcctttt 9120
   ccaaagctgc atcttattca gctattcttt ctactcagaa tgactttctc tatctggtat 9180
   attcttgctt actcttcaag gcccagacaa atgtcgtttc ctctattaag ctttccctga 9240
   tcctcaccct tggacaaaat taatagcttc tacccttttt tcatatgcag tcatataagc 9300
   actaaaagat aatgtataca ttctattaaa ttaattgcat atatgtttta attttttcac 9360
   ttcattcata aactttggaa gcagcagttg attcttcata tcagcactct tttctttcat 9420
   aatatctaat tattttaggt cataatagtc ctcgttaaat gtttggatta aatctaattg 9480
   aattgaattc aagtgccaga tctctgtaat atgtctacca tgcaactcta ctaccctaag 9540
   gttttgtttg tctgtttgtt tgtttttatt tttttgagac agctttaaca cctaggctgg 9600
   agtgcagtgg tgcaatcttg gctcactgca acctccacct cccaggctca accattcctc 9660
   ccacctcctg agtagctggg actacaggtg cataccacca tgcccggcta atttttttgt 9720
   attgtctgta gagacagggt ttcgccatgt tgtccaggct ggtgtctaac tcctgggctc 9780
   aggcaatctg cccacctcag catcccaaag tgctgggatt acaggtgtga gccactgctg 9840
   ccagtcaact ctgagttatg agacagattt gaggactaat tgaaaagcta acttccctgg 9900
   gacatccagg gtttttataa taaaaggatc accaaggctg aatattttat aaggaactca 9960
   gcctaaaggt tttggatagt gcacacccac tccttgccag ggcattagct gctcaagaag 10020
   cagagtgttc tggactggat aggcaatatc cttacttaca tatactacaa tacaattctg 10080
   atgctaaccc cccaaattag tgtcagaccc tacaggttaa gagcatggtt cccaaccaga 10140
   ttgccctcac tttaaatgcc agctacaagt tcagggtacc ccagaccact cacatttctg 10200
   acaaactgcc tgcaatctta aggattccca tgaccctcct cattgataat ttgctagaat 10260
   aactcacaga atttaagaaa gtgctgtact tcctatttca gttttattaa aaaataaaat 10320
   aaaatcagaa ccagctaaat gaaaagggca aggtctagga gggtcccgaa cacagagcct 10380
   ccgtgccctc tccccgtgga attagaacac atcgccccca gcactctccc acactctgcc 10440
   agcacattga tgggttcacc aaccaggaag ctccaccaag tttttattga ggtatcttta 10500
   cataggcatg attgattgaa tcattggccg cttgactgat ctcaatctct aggatccctt 10560
   cctggggctg ataccactag tttcaaagct gcaatcctct taccatatgg ttggtcttga 10620
   ccagcaccat cctgagtcat tgccatgcat aaactcaggt atggtctaag gatccaccac 10680
   agataacaaa gccactcctg tcactcatga aattccaacg gttagaaaca ccctcccagg 10740
   ataccaggac aaagatgaga caaattgttt attatcacct tgaattcaat tgagtgattt 10800
   agtctacaat ccggaaaact aagtatagat actaccattt tcatggattt ggatctttct  10860
   tcatcttggc ctcaaataac catggaaata cttcagggca tctgaacaac tccatgccca  10920
   gctaatactc tatctctctt ctgtctttac aggtactttg gcccagtcaa tcaaaggtag  10980
   gagaaatggc ttctttctat actcagactc agaatattga cggaaatttg gcttcctaca  11040
   acagtagtcc tacaggagcg aacagtttag aatgaatgaa atgacgggga tagagaggtg  11100
   atgtctctat tgtcaaccaa atcagtgacc tgacataacc tgttccgggc agcttgcctg  11160
   tagctaagca tttaactggt ctcttacagg agaagcagga ccctagtagc tagggacaca  11220
   tctcaaactg tgacccatga accagtaggt attggcgtca cctgggatcc tgacgttagt  11280
   ctctgtcaat ccttctcttt agttcatcta ttctacccaa agtgatctca tcatctggta  11340
   tgctgttagc agtttcttac ctgtatagta tcttccaaat aacatgcccc aaaatcccaa  11400
   agttttaccc ctactaatta cagcaatgtc tcttttattc ttcaccccct gacgcagata  11460
   ttggcgtcac ccgagagcat gttagtaatg cagaatctcc cctccccaga actactaaat  11520
   agcacctgaa attttaacaa gatccccatg tgattcatgt gcacatcaaa gtttgagaaa  11580
   cactactcta atgatctcct ggtatgcaga agcagggaga atttcagagg caagatcctt  11640
   aatagaacca cggcttttct catttcagga aaccacttgg ttaaggtgta tgactatcaa  11700
   gaagatggtt cggtacttct gacttgtgat gcagaagcca aaaatatcac atggtttaaa  11760
   gatgggaaga tgatcggctt cctaactgaa gataaaaaaa aatggaatct gggaagtaat  11820
   gccaaggacc ctcgagggat gtatcagtgt aaaggatcac agaacaagtc aaaaccactc  11880
   caagtgtatt acagaagtat gtaatcccct ttggtctgtt tgttgtgaaa ttaatcagta  11940
   tttgctgttc tggtgagctt tttatctggg gtgaaagtgg aaatagatcc tcaacagtaa  12000
   tattatcgcc tgttctctta atttcagctt gcctctttta aaatactgta agatacttcc  12060
   ctcaccctat tgaaaaacta cagccagtcc tgtaaaattt tgtttacctt tgggtgggct  12120
   ccatggattc aagcaattca gcactgagtt gaatgaaggg gttggggaga cagaggtgat  12180
   cataaggtga gcagcagtga gaactggagc gcagtggagc atgaagagta tctagtcttc  12240
   cttctgttcc agatacttcc tggtagttag actacatggg cttccccagg aatcctgggg  12300
   gacttaagag catcaagatg cattgagttt tggcgcaagg atcttccctt gccctgccac  12360
   ccacagagga aaagcctgct gccctccaca gcggcattat tgcagacagg caggagaaaa  12420
   cgaaccagga aaaacaactt tcgcaacctg aaggtttgtc tctccttttc cctacagtgt  12480
   gtcagaactg cattgaacta aatgcagcca ccatatctgg ctttctcttt gctgaaatcg  12540
   tcagcatttt cgtccttgct gttggggtct acttcattgc tggacaggat ggagttcgcc  12600
   agtcgagagg taaaagaatg ctcttagatg agagatggga ccacctgaga ccctcagctt  12660
   tcctcctacc attatgtacc caatggaaga gactgagttg gtgcttcttg ctagtgtgca 12720
   tagttgggtg aggctgtatt tctctgagac aggagaaagg atacttggtg ttatcacagc 12780
   atgttcacct gtctcaagat acagctccct ctgtgaaaaa aaaaaaaaac aaaaacaggc 12840
   gcagtggctc acgcctgtaa tcacaacact ttgggaggcc gaggcgggca gatcacgagg 12900
   tcaggagatc gagaccatcc tggctagcac ggtgaaaccc cgtctttact aaaaatacaa 12960
   aaaattagcc gggcgtggtg gcgggtgcct gtagtcccag ctactctgga ggctgaggca 13020
   ggagaatggc atgaacccgg gaggcggagc ttgcgtgagc cgagatcgtg ccactgcact 13080
   ccagcctggg cgacagagca agactccgtc tcaaaaaaaa aaaaaaaaaa aactatttta 13140
   agtaaaccct tacatacaga aaagtgaatc acaagcatac agctcaaaaa aaaaattcac 13200
   agagcaaata cacccatgca gccagcacat aatttaagaa agacaatatt accaatgcca 13260
   ttgaagcccc tcttgtggtc acttccagcc actacccatc ctcaagagtg agcattgtcc 13320
   tgacttctaa caacaaggat taactttgct tgtttttgtc ctttatgtaa atggactcat 13380
   atactaagta ttcttgtgag tagcttcttt cactcaacaa catgtttatg tgctttatcc 13440
   atactcttgt gtatatataa aactcttatt gttatttagt attccatttt gtgaatataa 13500
   cccaatttat tatgcattct accttgtgtg gattctgttt cttttttgtg cagcttcaga 13560
   caagcagact ctgttgccca atgaccagct ctaccaggta aggggatgaa gaataaaaga 13620
   gacattgctg taattagtgg gggtaaatct ttgggattga ggggcatgtt atttggaaga 13680
   tcctatacag gtaagaaact gctaacagca taccagatga tgagatcagt ttgggtagaa 13740
   taaatgaact aaagagaagg attgacagag actgaggtca ggatctagtg agcacaagtt 13800
   gaagaacaca ctgagaggga cacacgaaga aactctcgac aggctgggca ctcactatag 13860
   acaggccatg gctcatgcct gtaatcccag cactttggga agccaaaaca ggtgaatcac 13920
   ctgaggtcag gaattcgaga caagcctggc caacatgatg aaactctgtc tctactaaaa 13980
   atacaaaaaa aattaaccag gcgtggaggc gcgcgcctgt agtcccagct actcaggagg 14040
   ctgaggcagg aggaccactt gaacccagga ggtcgaggtt gcagtgagct gcgattgtgc 14100
   cactgcactc cagcctgggc aacagagaaa gactccgtct caaaaaaaaa aaagagagag 14160
   agagaaaaag aaaaaagaca gagcctccat ctccttgtcc tctttccatc ctcaggacca 14220
   tgaagtaccc actccaaatt ctcacatata aaaaacattc aataaacatg catcaaatta 14280
   attaatagag gatggaaaaa atgacttatg actgtgctgt cctttccagc ccctcaagga 14340
   tcgagaagat gaccagtaca gccaccttca aggaaaccag ttgaggagga attgaactca 14400
   ggactcagag taggtgggtt cttcaatgcc aattctaata aaggaccctt gcatcaactg 14460
   ccctcgcaat tgcttctaag tctagctccc ttccctaagc ggctataagc atcagactct 14520
   ggggatcagg gattgggacg tggtttgggg tactcttttc taaaaattct ggggccatac 14580
   tgattgtctt ggcctaggta aatatgaatt ttatgtatct gtaaatcctg tcagagcagg 14640
   gcctcaagcc atagagatgc tgaatattaa tcttaaccta catttgaatt tctcattatc 14700
   tacactatta acattttggg ctaattaatt atttgtgatg aggggctagc ctgtgcattg 14760
   taggagttat ggaagcatcc ctggcctctc tccaccagat gctggtagat tgtccagtgt 14820
   gacaatcaaa aatgtgtcca gacattacca aatgtgtcca aacatcacct ccagggcaaa 14880
   atcaccctta gttaagaacc actaacccat attaaccttc caatcaataa atcaatcagt 14940
   cagaagttat gatttaatta atctatctga agtttctatc aggaagacag ggttgaaagc 15000
   attatttgtt ttttttgaac aaattgcaat ttttcttttt tcagtccagg tgttctcctc 15060
   ctattcagtt cccagaatca aagcaatgca ttttggaaag ctcctagcag agagactttc 15120
   agccctaaat ctagactcaa ggttcccaga gatgacaaat ggagaagaaa ggccatcaga 15180
   gcaaatttgg gggtttctca aataaaataa aaataaaaac aaatactgtg tttcagaagc 15240
   gccacctatt ggggaaaatt gtaaaagaaa aatgaaaaga tcaaataacc ccctggattt 15300
   gaatataatt ttttgtgttg taatttttat ttcgtttttg tataggttat aattcacatg 15360
   gctcaaatat tcagtgaaag ctctccctcc accgccatcc cctgctaccc agtgaccctg 15420
   ttgccctctt cagagacaaa ttagtttctc tttttttttt tttttttttt ttttttgaga 15480
   cagtctggct ctgtcaccca ggctgaaatg cagtggcacc atctcggctc actgcaacct 15540
   ctgcctcctg ggttcaagcg attctcctgc ctcagcctcc cgggcagctg ggattacagg 15600
   cacacactac cacacctggc taatttttgt atttttagta gagacagggt tttgctctgt 15660
   tggccaagct ggtctcgaac tcctgacctc aagtgatccg cccgcctcag cctcccaaag 15720
   tgctgggatt acaggtgtga gccaccatgc ctggtcttaa aaccagtttc ttatatatct 15780
   ctctggaggt attctaggca tatatgagca cattctcaag tacatattat cctcccttcc 15840
   cctatctttt agacaaatga tatcaaacta tacatcttgt gagattattg cataccatta 15900
   tatgaagata ccattatatc ctttttaatg caaccatatt gtacaaatag actatgattt 15960
   atttaacctg ttatctatca gtggatattt aagttggtag ttggttccaa tcttttgctc 16020
   ttacaacaat tctgcaatga ctaacattgt ataaatatca tttttaaaaa taattgcatt 16080
   gaagcataat gtacatgcca taaaatccac ccatcttaag tgatttcacc tgttctcaga 16140
   aatttttagt aaatttaact aattgtacag ccattaccat aatccagctt taggacattt 16200
   tctttttttt cttttctttt cttttttttc tttttttttt ttttttgaag tggaatcttg 16260
   ctctgtggcc caggctggag tgcagtggcg cgatctcagc tcactgcaac ctccacctcc 16320
   tgggttcaag cgattctctt gccttggcct cccgagtagc tgagactaca ggcacatgcc 16380
   accacgccca gctcattttt tgtgtattta gtatttgtgt atctagtatt tgtgtactta 16440
   gtagagacag ggtttcacca tgttggccag gctggtctcc aattcctgac ctcaggcgat 16500
   c 16501
<210> 2
   <211> 50
   <212> DNA
   <213> Homo sapiens
<400> 2
   agctgctgca caggctggct ggctggctgg ctgctaaggg ctgctccacg 50
<210> 3
   <211> 50
   <212> DNA
   <213> Homo sapiens
<400> 3
   cggaaaaaca aaaggcatct gcacctgcag ccctgctgag gcccctgctg 50
<210> 4
   <211> 50
   <212> DNA
   <213> Homo sapiens
<400> 4
   acccaggctg atagttcggt gacctggctt tatctactgg atgagttccg 50
<210> 5
   <211> 50
   <212> DNA
   <213> Homo sapiens
<400> 5
   tggaacatag cacgtttctc tctggcctgg tactggctac ccttctctcg 50
<210> 6
   <211> 435
   <212> DNA
   <213> Homo sapiens
<400> 6
   ttattccacc tattaccttc caaacgcctc tttcataatc tcaaaaaaat aataaaacca      60
   aataccgtat ctcacgccta taatcccaac actttaaaaa actaaaacaa ataaatcaca     120
   aaatcaaaaa ttcgaaacca acctaaccaa caaaataaaa ccccgtctct actaaaaata     180
   caaaaattaa ccgaacttac taacacgcac ctataatctc aactactcaa aaaactaaaa     240
   caaaaaaatc gcttaaaccc cgaaaataaa aattacaata aactaaaata acgccactac     300
   actccaacct aaacaacaaa ataaaactct atctcaaaaa aaaaaaaaaa aaaaaaaata     360
   aaaaaaattc ctaaataaaa acctaaacta aaataacttt ccatttaaaa atccaacccc     420
   aaacatctaa aaatc                                                      435
<210> 7
   <211> 438
   <212> DNA
   <213> Homo sapiens
<400> 7
   ataaacctca ctcccatcaa taatatccct ctcaaatccc ttcccccacc cacctaaaat      60
   aaccttacct tacgaaaaaa aaataaccaa taccaaacca aaaaaaaacg tactatattc     120
   catctcccaa cgaaactcat ccaataaata aaaccaaatc accgaactat caacctaaat     180
   aaaaactacc ctcccctaac taactcacaa ataccgaaaa aaaaaaaata aaaaaaaaac     240
   taaaaaatat ctacttctct actttctacc gttaataata aaaaattttc aaaataaaaa     300
   taaactaaaa ataaaataaa aaaaaaacga tataaattac tctatctcta aacaaaaaac     360
   taacaaaaaa tataaaaaaa ataacctaaa ctttaacgtc cctattaaca atacaaccac     420
   atttacacac aacctaaa                                                   438
<210> 8
   <211> 472
   <212> DNA
   <213> Homo sapiens
<400> 8
   aatccctcct aaattcatta cctacaacaa caacaactaa caacaaataa caactaacta 60
   cgatcctaac aactaataac aaaaacattt atctccctca taaaaaaacg atcccaaaac 120
   catctcccac ccaacatcca ttacgattcc ctatacaaaa taaatctcta aataaaaatc 180
   caacactctc tccctcttct tccccaccac cttcaccctc cttaacgaaa aaacaaaaaa 240
   catctacacc tacaacccta ctaaaacccc tactactcac acttacaaca aaaaataaaa 300
   actctaaatt cttaccttct ctcaaaaacc ccaaccccaa caataataaa taaaaccaat 360
   ctaactacta cacaaactaa ctaactaact aactactaaa aactactcca cgcttttacc 420
   gaaaaacaaa aactaacata aaacaaaaaa aaaacctaac tatcctcatc ct 472
<210> 9
   <211> 50
   <212> DNA
   <213> Homo sapiens
<400> 9
   agtcatctgt tttgcttttt ttccagaagt agtaagtctg ctggcctccg 50
<210> 10
   <211> 50
   <212> DNA
   <213> Homo sapiens
<400> 10
   cgcccagcct cgctgttctt atcttggcag cagattccga atgtcggctg 50
<210> 11
   <211> 50
   <212> DNA
   <213> Homo sapiens
<400> 11
   cgctcatcag gacttcagca ctgcccgtcc atggggacgt ctgcactcac 50
<210> 12
   <211> 50
   <212> DNA
   <213> Homo sapiens
<400> 12
   cgccttgagc tgtacctggc acatctttgt tgctcagcaa agagtggttg 50
<210> 13
   <211> 50
   <212> DNA
   <213> Homo sapiens
<400> 13
   tcttcccatc tgcgtcttgg ttaaggactt ttatcgtatc ctgtttatcg 50
<210> 14
   <211> 50
   <212> DNA
   <213> Homo sapiens
<400> 14
   aagatctgct ggaggccagg cacagacagg tgctttaact caagttatcg 50
<210> 15
   <211> 50
   <212> DNA
   <213> Homo sapiens
<400> 15
   agtaactgca gcaatgagtg cccgggctgt gcttggagta ccagtgctcg 50
<210> 16
   <211> 50
   <212> DNA
   <213> Homo sapiens
<400> 16
   cgccccaagt actaagtggc acagcttggt gagcaaggac atttttattg 50
<210> 17
   <211> 50
   <212> DNA
   <213> Homo sapiens
<400> 17
   cggcaagatg aaacaagctt attaggctct gtcttttaag ggcataccag 50
<210> 18
   <211> 50
   <212> DNA
   <213> Homo sapiens
<400> 18
   cgcaacacag ggctgcctcc cccggtatat gggccccact ccacagaggt 50
<210> 19
   <211> 50
   <212> DNA
   <213> Homo sapiens
<400> 19
   cggcagagca cacagctgca gaagtaaaaa ggattgaaac atttggatcc 50
<210> 20
   <211> 50
   <212> DNA
   <213> Homo sapiens
<400> 20
   acccaggctg atagttcggt gacctggctt tatctactgg atgagttccg 50
<210> 21
   <211> 50
   <212> DNA
   <213> Homo sapiens
<400> 21
   tggaacatag cacgtttctc tctggcctgg tactggctac ccttctctcg 50
<210> 22
   <211> 50
   <212> DNA
   <213> Homo sapiens
<400> 22
   agctgctgca caggctggct ggctggctgg ctgctaaggg ctgctccacg 50
<210> 23
   <211> 50
   <212> DNA
   <213> Homo sapiens
<400> 23
   cggaaaaaca aaaggcatct gcacctgcag ccctgctgag gcccctgctg 50
<210> 24
   <211> 50
   <212> DNA
   <213> Homo sapiens
<400> 24
   agtcatctgt tttgcttttt ttccagaagt agtaagtctg ctggcctccg 50
<210> 25
   <211> 50
   <212> DNA
   <213> Homo sapiens
<400> 25
   cgctgcagtc agactccaaa gtcaggaacg tgagggctac catctctcaa 50
<210> 26
   <211> 50
   <212> DNA
   <213> Homo sapiens
<400> 26
   cgaaggcccc cagctgtggg agcccacgct atttattggt gatcaaagaa 50
<210> 27
   <211> 50
   <212> DNA
   <213> Homo sapiens
<400> 27
   cgcaggcact tacgatcctg ccgtcgccag agcccagatc caccgttttt 50
<210> 28
   <211> 50
   <212> DNA
   <213> Homo sapiens
<400> 28
   gggctcccgg gctgggcagg taaggagcgc tggtattggg ggcgcaggcg 50
<210> 29
   <211> 50
   <212> DNA
   <213> Homo sapiens
<400> 29
   ttccgcccac aattttctga taacagcctt caaggcctca gttgctgtcg 50
<210> 30
   <211> 50
   <212> DNA
   <213> Homo sapiens
<400> 30
   cgggaccctg ttgctgactg ctcaagagga ggcaagctgg atctctctta 50
<210> 31
   <211> 50
   <212> DNA
   <213> Homo sapiens
<400> 31
   taaatgatgt cttggggctg tggcccgagc tgcctcaggt agatcccacg 50
<210> 32
   <211> 50
   <212> DNA
   <213> Homo sapiens
<400> 32
   ggggactgtg ggtcctctta ggggctgtga cgctgctatt tctcatctcg 50
<210> 33
   <211> 50
   <212> DNA
   <213> Homo sapiens
<400> 33
   cgcactcagc aaggcctcct gccctgagag aggctccgcc cactaccccc 50
<210> 34
   <211> 50
   <212> DNA
   <213> Homo sapiens
<400> 34
   ctcatgaagc aaggtctgcc tctacttctg tgatgtggtt catgtttccg 50
<210> 35
   <211> 50
   <212> DNA
   <213> Homo sapiens
<400> 35
   gacctttagc ctcaaactcc cgtggtggaa acagttagga ttggtggacg 50
<210> 36
   <211> 50
   <212> DNA
   <213> Homo sapiens
<400> 36
   gagagacaat acccttccaa aatacacttc aacataaagt tttcttttcg 50
<210> 37
   <211> 50
   <212> DNA
   <213> Homo sapiens
<400> 37
   cggtatacct gccctacttc ggtatagtcc ttggcacatg gcaggcactc 50
<210> 38
   <211> 50
   <212> DNA
   <213> Homo sapiens
<400> 38
   atggtagccc cttctgcggg gagcacacaa cagtcttcag ttcttctgcg 50